(19) Europäisches Patentamt / European Patent Office / Office européen des brevets

(11) **EP 4 218 652 B1**

(12) **EUROPEAN PATENT SPECIFICATION**

(45) Date of publication and mention
of the grant of the patent:
**25.03.2026 Bulletin 2026/13**

(21) Application number: **20956605.8**

(22) Date of filing: **03.12.2020**

(51) International Patent Classification (IPC):
*A61B 34/37* (2016.01)     *A61B 34/30* (2016.01)
*A61B 90/00* (2016.01)     *A61B 34/20* (2016.01)
*A61B 34/00* (2016.01)

(52) Cooperative Patent Classification (CPC):
**A61B 90/361; A61B 34/37;** A61B 34/25;
A61B 2034/2059; A61B 2090/365; Y02P 90/02

(86) International application number:
**PCT/CN2020/133490**

(87) International publication number:
**WO 2022/073290 (14.04.2022 Gazette 2022/15)**

(54) **SURGICAL ROBOT, AND GRAPHICAL CONTROL DEVICE AND GRAPHIC DISPLAY METHOD THEREFOR**

CHIRURGISCHER ROBOTER UND GRAFISCHE STEUERUNGSVORRICHTUNG UND GRAFISCHES ANZEIGEVERFAHREN DAFÜR

ROBOT CHIRURGICAL, ET DISPOSITIF DE COMMANDE GRAPHIQUE ET PROCÉDÉ D'AFFICHAGE GRAPHIQUE ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.10.2020 CN 202011068091**

(43) Date of publication of application:
**02.08.2023 Bulletin 2023/31**

(73) Proprietor: **Shenzhen Edge Medical Co., Ltd.
Shenzhen, Guangdong 518000 (CN)**

(72) Inventors:
• **GAO, Yuanqian
Shenzhen, Guangdong 518000 (CN)**
• **WANG, Jianchen
Shenzhen, Guangdong 518000 (CN)**

(74) Representative: **Zaboliene, Reda
Metida
Business center Vertas
Gyneju str. 16
01109 Vilnius (LT)**

(56) References cited:
EP-A1- 3 115 159          WO-A1-2010/151438
WO-A1-2018/236587     WO-A2-2008/002830
CN-A- 102 243 764        CN-A- 109 419 555
CN-A- 110 236 682        CN-A- 111 991 084
CN-A- 111 991 085        US-A1- 2014 055 489
US-A1- 2020 237 456

**Description**

The present application claims priority to CN Patent Application No. 202011068091. 4, filed on October 8, 2020, in China National Intellectual Property Administration, entitled "surgical robot, and graphical control device and graphic display method therefor".

**FIELD**

The subject matter herein generally relates to surgical instrument, in particular to a surgical robot, a graphical control device, and a graphic display method.

**BACKGROUND**

Minimally invasive surgery refers to a surgical method of performing a procedure in a human body cavity using modern medical instruments such as laparoscopes, thoracoscopes, and so on. Compared with traditional surgical methods, minimally invasive surgery has advantages of being in little trauma, little pain, fast recovery, and the like.

With advances in science and technology, minimally invasive surgical technologies are becoming mature and widely used.

Invasive surgical robots usually include a master console and a slave operating device. The slave operating device includes a plurality of operating arms. The operating arm includes a camera arm with an image end instrument and a surgical arm with an operating end instrument. The master includes a display and a handle. The doctor operates the handle to control the motion of the camera arm or surgical arm under the vision field provided by the camera arm on the display.

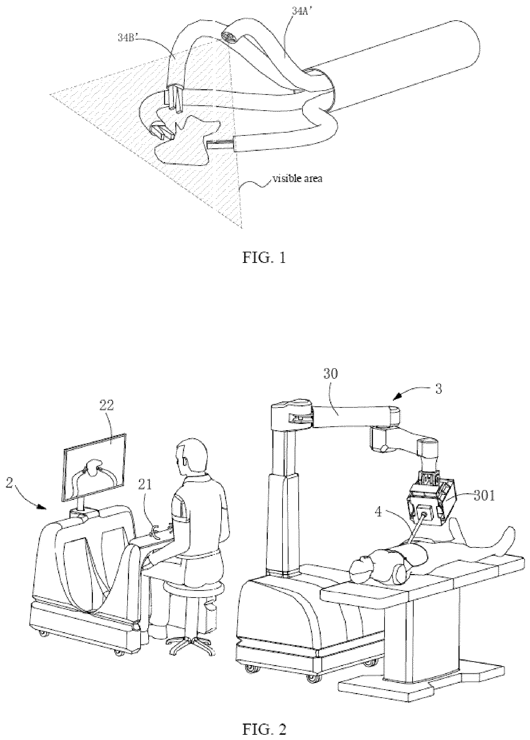

However, in most cases, as shown in FIG. 1, the vision field provided by the camera arm 34A' can only observe local area of the surgical arm 34B', of which the observed area is regarded as a visible area, so there are large invisible areas. It may cause surgical problem if the doctor cannot observe the motion of the camera arm 34A' in the visible area, or a happened collision or a potential collision between surgical 34B' and camera arm 34A' in the invisible area. D1 (EP3115159A1) discloses a medical robotic system including an entry guide with surgical tools and a camera extending out of its distal end. To supplement the view provided by an image captured by the camera, an auxiliary view including articulable arms of the surgical tools and/or camera is generated from sensed or otherwise determined information about their positions and orientations and displayed on a display screen from the perspective of a specified viewing point. Intuitive control is provided to an operator with respect to the auxiliary view while the operator controls the positioning and orienting of the camera. D2 (WO2010151438A1) discloses a medical robotic system includes an entry guide with surgical tools and a camera extending out of its distal end. To supplement the view provided by an image captured by the camera, an auxiliary view including articulatable arms of the surgical tools and/or camera is generated from sensed or otherwise determined information about their positions and orientations are displayed along with indications of range of motion limitations on a display screen from the perspective of a specified viewing point.

**SUMMARY**

Based on this, it is necessary to provide a surgical robot, and a graphical control device, and a graphic display method to help a doctor observing a motion state of the operating arm in all orientations.

The invention is defined in appended independent claims 1,13 and 14, further embodiments are described in the dependent claims.

In one aspect, a surgical robot is provided. The surgical robot includes: an input portion; a display; an operating arm having a plurality of joints and sensors that each sense the joint variable, a feature point sequence consisting of a plurality of feature points, the feature points are arranged orderly, and each of the joints is associated with at least one of the feature points; and a controller. The controller is coupled to each of the input portion, the display, and the sensors, and configured to: obtain the feature point sequence of the operating arm and a kinematic model corresponding to the feature point sequence; obtain joint variables sensed by each of the sensors, and obtain a virtual camera by the input portion; determine a projection point of each of the plurality of feature points in the feature point sequence on a projection plane of the virtual camera according to the kinematic model and the joint variables; orderly fit and connect the projection point of each of the plurality of feature points to generate a projected image of the operating arm; and display the projected image on the display.

When determining the projection point of each of the plurality of feature points in the feature point sequence on the projection plane of the virtual camera according to the kinematic model and the joint variables, the controller is configured to: acquire a first position of each of the plurality of feature points of the feature point sequence in a reference coordinate system based on the kinematic model and the joint variables; convert the first position each of the plurality of feature points to a second position in a coordinate system of the virtual camera; acquire a virtual focal length of the virtual camera and determine the projection plane of the virtual camera based on the virtual focal length; and acquire a projection point of the second position of each of the plurality of feature points on the projection plane based on the virtual focal length.

When determining the projection point of each of the plurality of feature points in the feature point sequence on the projection plane of the virtual camera according to the kinematic model and the joint variables, the controller is further configured to: acquire a first position of each of the plurality of feature point in a reference coordinate system based on the kinematic model and the joint variables; convert the first position of each of the plurality of feature points to a second position in a coordinate system of the virtual camera; acquire a contour information of each of the plurality of joints corresponding to the corresponding feature points which is associated with the joint; acquire a projection point of the second position of each of the plurality of feature points on the projection plane according to the virtual focal length and the contour information.

When fitting and connecting the projection point of each of the plurality of feature points orderly to generate the projected image of the operating arm, the controller is further configured to: fit and connect the projection point of each of the plurality of feature points orderly to generate the projected image of the operating arm according to the contour information.

When fitting and connecting the projection point of each of the plurality of feature points orderly to generate the projected image of the operating arm, the controller is further configured to: fit and connect the projection point of each of the plurality of feature points orderly to generate a projected image of the operating arm according to the order of the plurality of feature points corresponding to the projection points of the plurality of feature points in the feature point sequence.

When fitting and connecting the projection point of each of the plurality of feature points orderly to generate the projected image of the operating arm, the controller is further configured to: acquire an icon of an end instrument of the operating arm; determine a pose and position of the end instrument on the projection plane of the virtual camera based on the joint variables and the kinematic model; process the icon by rotating and/or zooming based on the pose and position of the end instrument on the projection plane of the virtual camera; and splice the icon with one projection point located at a distal end of the operating arm to generate the projected image.

When acquiring the icon of an end instrument of the operating arm, the controller is configured to: acquire a type of the operating arm, and match out an icon of the end instrument of the operating arm according to the type.

The virtual camera having a virtual focal length and/or a virtual aperture which is selectable, when determining the projection point of each of the plurality of feature points in the feature point sequence on the projection plane of the virtual camera according to the kinematic model and the joint variables, the controller is configured to: acquire the virtual focal length and/or virtual aperture selected by the input portion; and determine the projection point of each of the plurality of feature points in the feature point sequence on the projection plane of the virtual camera according to the virtual focal length and/or virtual aperture, the kinematic model, and the joint variables.

Before displaying the projected image on the display, the controller is further configured to: detect whether the projected image is distorted; when the projected image distortion is detected to be distorted, increase the virtual focal length of the virtual camera and repeat a process of determining the projection point of each of the plurality of feature points in the feature point sequence on the projection plane of the virtual camera according to the virtual focal length and/or virtual aperture, the kinematic model, and the j oint variables; and displaying the projected image on the display when the image is detected to be not distorted.

The controller is further configured to: acquire a position of the projection point of each of the plurality of feature points in a reference coordinate system; acquire a quantity of first projection points, wherein the first projection points are the projection points which fall within an edge area of the projection plane or an edge area of a display window, the display window being configured for displaying the projected image on the display; and calculate the ratio of the quantity of the first projection points to a total quantity of the projection points, and determine that the image is distorted when the ratio reaches a threshold.

The operating arm includes a camera arm with an image end instrument, and the controller is further configured to: acquire a camera parameter of the image end instrument of the camera arm, and calculate a visible area of the image end instrument according to the camera parameter, the camera parameter comprising a focal length and aperture; determine the pose and position of the image end instrument in a reference coordinate system based on the joint variables and the kinematic model of the camera arm; convert the visible area of the image end instrument to a visible area of the virtual camera based on the conversion relationship between the pose and position of the image end instrument and the pose and position of the virtual camera in the reference coordinate system; and calculate a boundary line of the visible area of the virtual camera on the projection plane, and display the boundary line in the projected image on the display.

The operating arm includes a camera arm with an image end instrument and a surgical arm with an operating end instrument; when orderly fitting and connecting each projection point of each of the plurality of feature points orderly to generate the projected image of the operating arm, the controller is further configured to: acquire an operating image of a surgical area captured by the image end instrument of the camera arm; identify a feature portion of the surgical arm from the operating image; match out an associated first feature point from the feature point sequence according to the identified feature portion; and fit and connect the projection points of each of the plurality of feature points orderly and mark a first projection point from the projection points associated with the first feature point, and mark a line segment connected to the first projection point to generate the projected image of the operating arm.

The feature point sequence includes an unmatched second feature point. After matching out the associated first feature point from the feature point sequences based on the identified feature portion, the controller is further configured to: acquire the unmatched second feature point; generate an image model of the feature portion according to the contour information, the joint variables, and the kinematic model of the feature portion corresponding to the second feature point; convert the image model to a supplementary image in a coordinate system of the image end instrument; splice the supplementary image with an image of the feature portion corresponding to the first feature point based on the sequence of the first and the second feature points in the feature point sequence to form a complete sub-image of the operating arm in the operating image; and display the operating image with the complete sub-image of the operating arm on the display.

The controller is further configured to: acquire a maximum range of motion in a first orientation of the operating arm; calculate an amount of the motion in the first orientation of the operating arm based on the joint variables and the kinematic model; generate an icon based on the maximum range of motion and the amount of the motion in the first orientation; and display the icon on the display.

The first orientation includes forward and backward feed orientation.

The icon can be either a progress bar or a pie chart.

The controller is configured to increase or decrease the amount of movement, the color depth will vary accordingly with the variation of the progress bar.

The controller is configured to detect the controlled first operating arm, and mark the first operating arm on the projected image.

A plurality of selectable virtual cameras for input portion having difference poses and positions in a reference coordinate system.

The poses and positions of the plurality of virtual cameras in the reference coordinate system are determined by a reachable workspace of the operating arm in the reference coordinate system.

The poses and positions of the plurality of virtual cameras in the reference coordinate system are determined by a union space of the reachable workspace of the operating arm in the reference coordinate system.

The positions of the plurality of virtual cameras in the reference coordinate system remain outside of the union space, and the poses of the plurality of virtual cameras remain viewing towards the union space.

Each of the plurality of virtual camera has an selectable virtual focal length, the positions of the plurality of virtual cameras are located outside of a first area, the first area is a shortest area determined by the union space visible from the virtual focal length perfectly.

The positions of the plurality of virtual cameras are located inside of a second area, the second area is a longest area determined by the union space visible from the virtual camera perfectly.

The poses and positions of the plurality of virtual camera remain towards the center of the union space.

The controller is configured to display the projected image on a first display window of the display, and to generate a plurality of icons of the plurality of virtual cameras.

The plurality of icons has a fix position relative to the projected image, and is configured to move with a change of a viewpoint of the projected image.

The plurality of icons numbers six, and is configured for virtual imaging of the operating arm from left, right, up, down, front and back sides to generate the projected image under a corresponding viewpoint.

The plurality of icons is represented as either an arrow or a camera, and is configured to rotate to any position corresponding to the plurality of virtual cameras.

The plurality of icons is represented as a rotatable sphere, and is configured to rotate to any position corresponding to the plurality of virtual cameras.

When obtaining the virtual camera selected by the input portion, the controller is further configured to: obtain the virtual camera selected by the input portion and at least two target positions of the virtual camera input by the input portion; determine a target projection point of each of the plurality of feature points in the feature point sequence of on a projection plane of the virtual camera at each of the target positions, according to a preset speed of the virtual camera, the kinematic model, and the joint variables; fit and connect the target projection point of each of the target positions orderly to generate a target projected image of the operating arm; generate animation based on each target projected image; and play the animation on the display based on a preset frequency.

When obtaining the virtual camera selected by the input portion, the controller is further configured to: obtain a motion trace of the virtual camera input by the input portion; discrete the motion trace to acquire discrete positions of the virtual cameras, the discrete positions being target positions of the virtual camera; determine a target projection point of each of the plurality of feature points in the feature point sequence on the projection plane of the virtual camera at each of the target positions, according to a preset speed of the virtual camera, the kinematic model and the joint variables; fit and connect each of the target projection points of the target position orderly to generate a target projected image of the operating arm; generate animation based on each of the target projected images; and play the animation on the display based on a preset frequency.

The operating arm comprises a camera arm with an image end instrument, and the controller is configured to: acquire an operating image on a surgical area captured by the image end instrument; display the operating image on the display; display the projected image suspended on the operating image.

When the displaying the projected image suspended on the operating image, the controller is further configured to: acquire an overlapping region between the operating image and the projected image, and a part of a first image property in the overlap region; adjust a second image property in the overlapping region based on the first image property.

The controller is further configured to: mark at least partial of a first operating arm in the projected image and display the first operating arm on the display when the first operating arm of the operating arm reaches a threshold of an event.

The threshold is a warning threshold, the event is a situation to be avoided.

The warning threshold is based on at least one range of the motion of at least one of the plurality of joints in the first operating arm, the situation to be avoided is a limitation of a range of motion of at least one of the plurality of joints.

The warning threshold is based on a distance between the first operating arm and a second operating arm of the operating arm, the situation to be avoided is a collision between the first operating arm and the second operating arm.

The controller is configured to: obtain a minimum distance between the first operating arm and the second operating arm, and judge the relationship between the minimum distance and the warning threshold. When the minimum distance reaches the warning threshold and does not reach the situation to be avoided, performing a first identification to the minimum distance on the sub-image of the first operating arm and the second operating arm.

The controller is configured to: when the minimum distance reaches the situation to be avoided, perform a second identification to the minimum distance on the model of the first operating arm and the second operating arm.

When obtaining the minimum distance between the first operating arm and the second operating arm and thereby judging the relationship between the minimum distance and the warning threshold, the controller is configured to: constructing geometric models of the corresponding first and second operating arms according to the kinematic models and the structural features of the first and the second operating arm; discretizing the geometric models of the first operating arm and the second operating arm to obtain the external information point sets of the first operating arm and the second operating arm in a reference coordinate system; determining the minimum distance between the first operating arm and the second operating arm according to the external information point sets of the first operating arm and the second operating arm. Identifying the minimum distance on the sub-image of the first operating arm and second operating arm, which includes: determining the minimum distance point corresponding to the minimum distance and identifying the minimum distance on the model of the first operating arm and second operating arm.

The controller is configured to determine the direction of collision according to the position of the minimum distance point on the projected images of the first operating arm and the second operating arm in a reference coordinate system; mark the direction of collision between the first operating arm and the second operating arm on the projected image.

The surgical robot includes a mechanical handle coupled to the controller for controlling the operating arm, which configured to: generate a resistance against the movement of mechanical handle in the associated direction according to the direction of collision.

The mechanical handle includes a plurality of joint components, and a driving motor coupled to the controller for driving each joint component. The controller is configured to: generate a reverse torque in the associated orientation according to the resistance.

The controller is configured to: when the minimum distance is between the warning threshold and the situation to be avoided, the magnitude of the reverse torque is negatively correlated with the magnitude of the minimum distance.

In some aspect, the present application provide a graphic display method of a surgical robot, which includes: an input portion; a display; an operating arm having a plurality of joints and a sensor that sense the joint variables. A plurality of joints consists of locative degree of freedom and/or an oriented degree of freedom. The operating arm has a feature point sequence consisting of feature points arranged orderly, the feature points representing joint. The step of the control method includes: acquiring the feature points of the operating arm and a kinematic model thereof; acquiring joint variables sensed by the sensors, and a virtual camera selected by the input portion; determining a projection point of each of the plurality of feature points in the feature point sequence on a projection plane of the virtual camera according to the kinematic model and the joint variables; orderly fitting and connecting each projection point to generate a projected image of the operating arm; and displaying the projected image on the display.

In some aspect, the present application provides a computer-readable storage medium storing computer program. The computer program is configured to implement the step of loading and executing the graphic display method of any embodiment mentioned above.

In some aspect, the present application provides a graphical control device of a surgical robot, which includes: a storage for loading computer programs; and a processing unit for loading and executing computer programs. The computer program is configured to implement the step of loading and executing the graphic display method of any embodiment mentioned above.

The present application has the following beneficial effects:
by configuring the virtual camera to simulate the real camera to image the operating arm, it can realize the observation of all operating arm and each operating arm as a whole, which is helpful for the doctor to observe the motion state of the operating arm in all-round way, which in turn contributes to the reliability of the operation and continuity.

## DESCRIPTION OF DRAWINGS

FIG. 1 is a schematic partial view of the surgical robot in a surgical state of the present art;

FIG. 2 is a schematic structural view of the surgical robot according to an embodiment of the present application;

FIG. 3 is another schematic partial view of the surgical robot of FIG. 1according to an embodiment;

FIG. 4 is a flow chart of a graphic display method of a surgical robot according to an embodiment;

FIG. 5 is a schematic structural view of an operating arm and a power part of the surgical robot;

FIG. 6 is a schematic layout view of a virtual camera of the surgical robot of FIG. 1 according to an embodiment;

FIG. 7 is a schematic view of a configuration interface of a virtual camera of the surgical robot of FIG. 1 according to an embodiment;

FIG. 8 is a schematic view of projection imaging of the graphical display method of FIG. 4 according to an embodiment;

FIGS. 9 and 10 are schematic view of display interfaces of the graphical display method according to an embodiment;

FIG. 11 is a flow chart of a graphical display method according to an embodiment;

FIGS. 12 and 13 are schematic view of display interfaces of the graphical display method according to an embodiment;

FIG. 14 is a flow chart of a graphical display method according to an embodiment;

FIG. 15 is a schematic view of display interfaces of the graphical display method according to an embodiment;

FIGS. 16 and 17 are flow charts of a graphical display method of the surgical robot according to an embodiment;

FIG. 18 is a schematic view of display interfaces of the graphical display method according to an embodiment;

FIG. 19 is a flow chart of a graphical display method of the surgical robot according to an embodiment;

FIGS. 20 and 21 are schematic view of a configuration interface of the virtual camera of the surgical robot of FIG. 1 according to an embodiment;

FIGS. 22 and 23 are flow charts of a graphical display method of the surgical robot according to an embodiment;

FIG. 24 is a schematic view of a configuration interface of the virtual camera of the surgical robot of FIG. 1 according to an embodiment;

FIGS. 25 to 27 are flow charts of a graphical display method of the surgical robot according to an embodiment;

FIG. 28 is a schematic view of observing an operating arm with a large field of view;

FIG. 29 is a schematic view of display interface generated by adapting the large field of view as shown in FIG. 28;

FIG. 30 is a schematic view of display interface generated by adjusting the large field of view as shown in FIG. 28;

FIG. 31 is a flow chart of a graphical display method of the surgical robot according to an embodiment;

FIG. 32 is a schematic partial view of the surgical robot of FIG. 1 according to an embodiment;

FIGS. 33 and 34 are schematic views of display interfaces of the graphical display method according to an embodiment;

FIGS. 35 to 38 are flowcharts of a graphical display method of the surgical robot according to an embodiment;

FIG. 39 is a schematic partial view of a surgical robot according to another embodiment of the present application;

FIG. 40 is a schematic structural view of a graphical control device of the surgical robot according to an embodiment of the present application.

## DETAILED DESCRIPTION

For ease of understanding of the present application, the present application will be described more fully hereinafter with reference to the associated drawings. Preferred embodiments of the present application are set forth in the accompanying drawings. This application may, however, be embodied in many different forms and is not limited to the embodiments described herein, only by the scope of the appended claims. Rather, these embodiments are provided for the purpose of providing a more thorough and thorough understanding of the disclosure of the present application.

It should be noted that when an element is referred to as being "disposed on" another element, it may be directly on the other element or intervening elements may also be present. When an element is considered to be "connected" to another element, it may be directly connected to another element or intervening elements may be present at the same time. When an element is considered to be "coupled" to another element, it may be directly coupled to another element or intervening elements may be present at the same time. As used herein, the terms "vertical", "horizontal", "left", "right" and the like are intended for purpose of illustration only and are not intended to be limiting. As used herein, the terms "remote end" and "proximal end" are common terms in the art of interventional medical devices, where "remoted end" refers to the end far away from the user during the surgical procedure, and the "proximal end" refers to the end close to the user during the surgical procedure. As used herein, the terms "first/second" and the like refer to one component as well as a class of two or more components having common characteristics.

Unless defined otherwise, all technical and scientific terms used herein have the same meaning as commonly understood by one of ordinary skill in the art. The terminology used herein is for the purpose of describing particular embodiments only and is not intended to be limiting of the invention. As used herein, the term "and/or" includes all combinations of one or more of the associated listed items.

Referring to FIGS. 2 to 3, a schematic structural view of a surgical robot according to an embodiment, and a partial schematic view thereof are illustrated.

A surgical robot includes a master console 2 and a slave operating device 3 controlled by the master console 2. The master console 2 has a motion input device 21 and a display 22. A doctor gives a control command to the slave operating device 3 by operating the motion input device 21, to make the slave operating device 3 perform corresponding operation according to the control command of the doctor given to the motion input device 21, and the surgical area is observed by the display 22. In particular, the slave operating device 3 has an arm body mechanism, the arm body mechanism has a mechanical arm 30 and an operating arm 31 detachably mounted on a remote end of the mechanical arm 30. The mechanical arm 30 includes a base and a connection assembly sequentially connected, and the connection assembly has a plurality of joint assemblies. The operating arm 31 includes a link 32, a connection assembly 33, and an end instrument 34, in which the connection assembly 33 may have a plurality of joint assemblies, the operating arm 31 adjusts the pose and position of the end instrument 34 by adjusting the joint assemblies. The end instruments 34 may include an image end instrument 34A and one or more operating end instruments 34B. The image end instrument 34A is used to capture the image within the field of view, and display 22 is used to display the image. The operating end instrument 34B is used to perform surgical operation such as cutting, suturing.

The surgical robot shown in FIG. 1 is a single-port surgical robot, and each operating arm 31 is inserted into the patient's body through the same trocar 4 mounted at the distal end of the robotic arm 30. In a single-port surgical robot, the doctor generally only controls the operating arm 31 to complete basic surgical operations. At this time, the operating arm 31 of the single-port surgical robot should have both a positional degree of freedom(that is, a positioning degree of freedom) and a posture degree of freedom(that is, the orientational degree of freedom), so as to realize the change of the posture and pose and position within a certain range, for example, the operating arm 31 has the degree of freedom of horizontal motion x, the degree of freedom of vertical motion y, the rotation degree of freedom $\alpha$, the pitch degree of freedom $\beta$, and the yaw degree of freedom $\gamma$, the operating arm 31 can also be driven by the distal joint of the mechanical arm 30, that is, the power mechanism 301 to realize the degree of freedom of forward and backward motion (i.e., feed degrees of freedom) z. For example, the power mechanism 301 has a guide rail and a power part slidably arranged on the guide rail, and the operating arm is detachably mounted on the power part. On the one hand, the power part provides power for the joints of the operating arm 31 to realize the remaining 5 degrees of the freedom(ie,[ x, y, $\alpha$, $\beta$, $\gamma$]).

The surgical robot further includes a controller, which can be integrated to either the master console 2, or the slave console 3. The controller can also be independent to either the master console 2 or the slave console 3. For an instance, it can be deployed in the local or the in the cloud. Wherein the controller can be consisting of more than one processor.

The surgical robot further includes input portion, which can be integrated to either the master console 2 or the slave console 3. The input portion can also be independent to either the master console 2 or the slave console 3. For an instance, the input portion can be a mouse, a keyboard, a voice input device and a touch screen. In one embodiment, a touch screen disposed on the armrest of the main console 2, is used as the input part, and the information available for configuration can be displayed on the touch screen, such as the virtual camera to be selected and its virtual camera parameters. In other embodiments, the information available for configuration may be displayed on the display 22 of the main console 2 or other external displays.

The operating arm 31 also includes sensors configured to sense joint variables of the plurality of joints. These sensors include an angle sensor that senses the rotational motion of the joint assembly and a displacement sensor that senses the linear motion of the joint assembly, and an appropriate sensor can be configured according to the type of the joint.

The controller is coupled to these sensors and to the input portion and the display 22 of the main console 2.

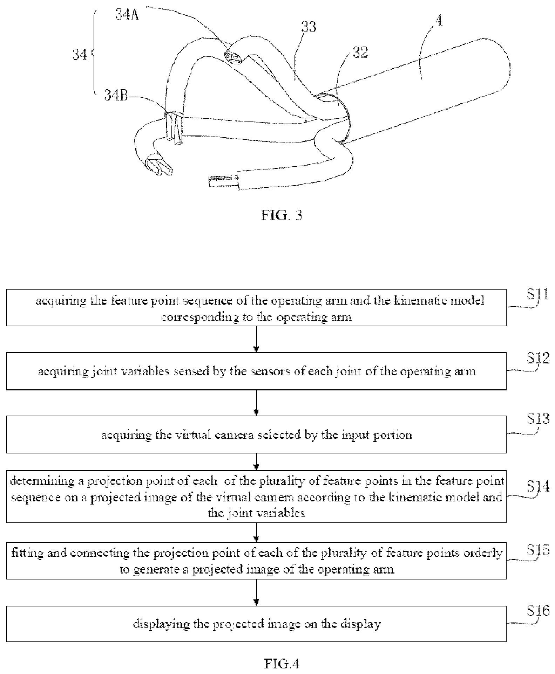

In one embodiment, a graphical display method of a surgical robot is provided, and the graphical display method can be executed by a controller. Referring to FIG. 4, the graphical display method includes the following steps:

In step S11, acquiring the feature point sequence of the operating arm and the kinematic model corresponding to the operating arm.

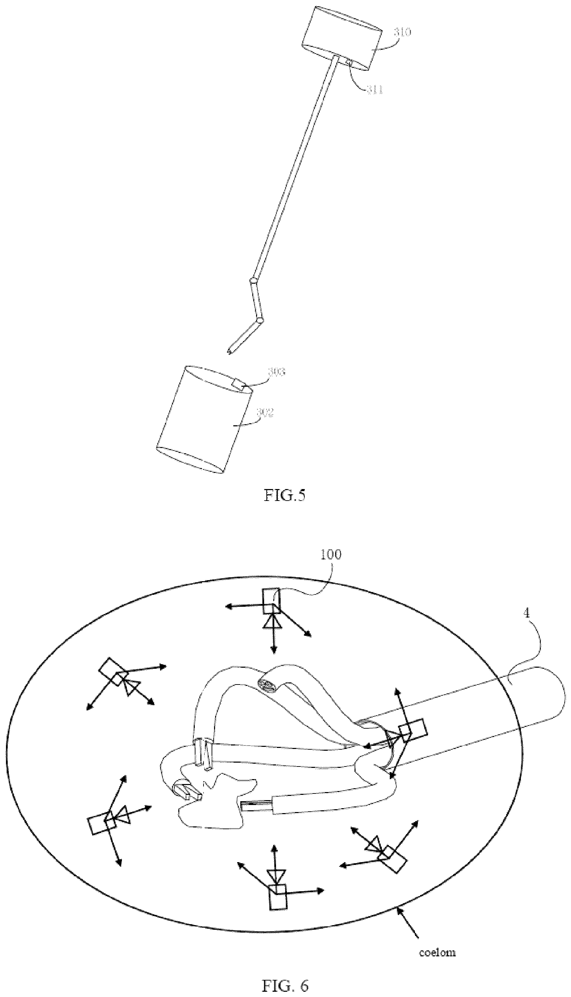

Exemplarily, as shown in FIG. 5, a storage unit 311 is mounted on the abutment surface of the driving box 310 of the operating arm 31 abutting against the power part 302 of the power mechanism 301, and correspondingly, when the power part 302 abuts the driving box 310, a storage unit 311 is installed. The abutting surface is provided with a reading unit 303 matched with the storage unit 311. The reading unit 303 is coupled to the controller. When the operating arm 31 is mounted on the power unit 302, the reading unit 303 communicates with the coupled storage unit 311, the reading unit 303 reads the relevant information from the storage unit 311. The storage unit 311 is, for example, a memory or an electronic tag. The storage unit stores, for example, one or a combination of two or more of the type of the operating arm, the feature point sequence, and the kinematic model pre-constructed according to the link parameters of the operating arm. The feature point sequence includes a plurality of feature points, the plurality of feature points can represent any feature part in the operating arm, and the feature part can refer to one or more of the end instruments, joints, and link of the operating arm.

For example, the storage unit 311 stores the feature point sequence and the kinematic model of the operating arm, and the required feature point sequence and the kinematic model of the operating arm can be obtained directly from the storage unit 311.

For another example, the storage unit 311 only stores the type of the operating arm while other storage units coupled to the controller store the feature point sequence and the kinematic model of different types of operating arm. The feature point sequence and the kinematic model of the corresponding operating arm can be obtained according to the acquired type of operating arm.

Step S12, acquiring joint variables sensed by the sensor of each joint of the operating arm.

The joint variable refers to the joint amount of the rotation joint and/or the joint offset of the mobile joint in the joint.

Step S13, acquiring the virtual camera selected by the input portion.

That is, a virtual camera is a non-existent camera, which does not actually capture the image of an object, but only reflects a concept of a viewpoint. As shown in FIG. 6, which is a spatial distribution showing a virtual camera relative to the operating arm. The default virtual camera 100 can be defined as any one of them. For example, the default selection is the virtual camera 100 on the puncture device 4. The parameters of the virtual camera can be configured, and the virtual camera parameters (i.e., configuration parameters) of the virtual camera include at least the (virtual) pose and position. The real camera includes camera parameters such as focal length and/or aperture, correspondingly the virtual camera parameters also includes virtual focal length and/or or virtual aperture. Typically, the (virtual) focal length corresponds to the field angle of the adjustable (virtual) camera, and the (virtual) aperture corresponds to the depth of field of the adjustable (virtual) camera. In one embodiment, the virtual camera parameters can also be described as including the field angle and/or the depth of field. For the virtual camera, the field angle and/or the depth of field are also virtual. Even if the camera, focal length, and aperture are virtual, imaging principles like real cameras can also be utilized to achieve the subject matter of the present application. Different virtual camera parameters can generate different imaging effects to doctors.

These virtual camera parameters can be solidified in a system configuration file stored in the memory of the surgical robot, and can be obtained by reading the system configuration file through the controller. These virtual camera parameters can also be manually set by the doctor through an input portion coupled to the controller before or during the operation, which is a method on demand. For example, these virtual camera parameters can be obtained by inputting related data through text control. For another example, these virtual camera parameters can be obtained by selecting from the option control.

The pose and position of the virtual camera can be the same as that of the real camera (i.e., the image end instrument) to observe the operating arm from the same viewpoint as the real camera. The pose and position of the virtual camera can also be different from the pose and position of the real camera, so that the operating arm can be observed from a different viewpoint than the real camera. Typically, the pose and position of the virtual camera can be selected to be different from the pose and position of the real camera for observation, which helps to obtain more comprehensive information of the operating arm. For example, the operating arm can also be a camera arm at this time, so that the virtual camera can observe it.

The acquired virtual camera includes acquiring the pose and position of the virtual camera and virtual camera parameters of the virtual camera.

In theory, the longest virtual focal length can be infinite, and the shortest can be infinitely close to 0. Exemplarily, imitating a lens of a real camera with a focal length ranging 2mm to 70mm to configure a selectable virtual focal length of the virtual camera, it can be configured as a virtual focal length of 2 mm to 50 mm, such as 2 mm, 5 mm, 10 mm, 20 mm. Further, the position of the virtual camera is configured according to the shortest virtual focal length and/or the longest virtual focal length. Wherein, the smaller the virtual focal length, the larger the projected image, and the more local details can be viewed; the larger the virtual focal length, the smaller the projected image, and the bigger image can be viewed.

In theory, the maximum virtual aperture can be infinite, and the minimum can be infinitely close to 0. Exemplarily, imitating a lens of a real camera with an aperture ranging F1, F1. 2, F1. 4, F2, F2. 8, F4, F5. 6, F8, F11, F16, F22, F32, F44, F64 to configure a selectable virtual aperture of the virtual camera, it can be configured as F2. 8, F4, F5. 6, F8. Wherein, the larger the virtual aperture, the smaller the depth of field; the smaller the virtual aperture, the larger the depth of field.

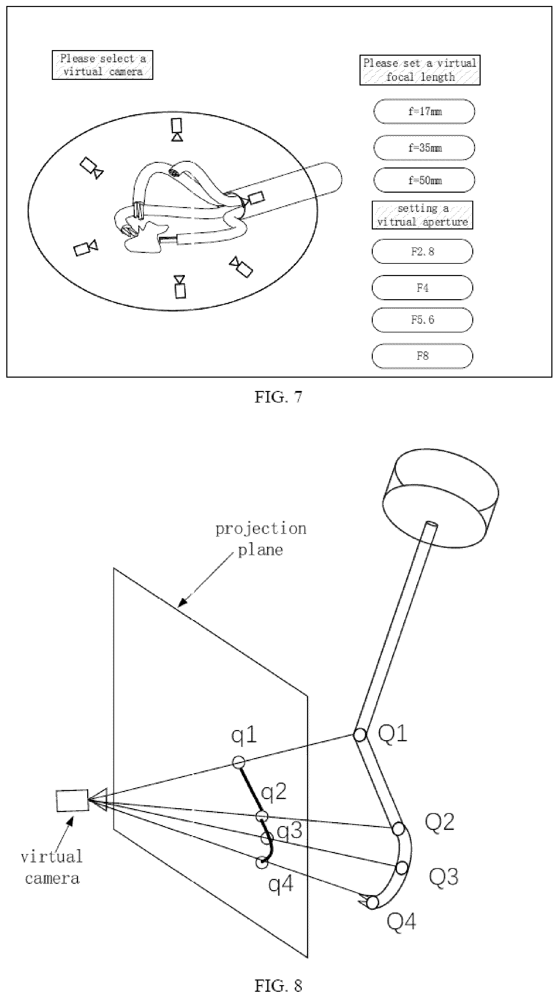

As shown in FIG. 7, a configuration interface of a virtual camera is illustrated. The pose and position, virtual focal length and virtual aperture of the virtual camera can be selected on this interface.

Step S14, determining a projection point of each of the plurality of feature points in the feature point sequence on the projection plane of the virtual camera according to the kinematic model and the joint variables.

For example, firstly, calculating the first position of each feature point in the feature point sequence in a reference coordinate system according to the kinematic model of the operating arm and the joint variables; secondly, converting the first position to be a second position in the virtual camera coordinate system according to the relationship between the coordinate system of the virtual camera and the reference coordinate system; and finally, taking the third position projected by the second position on the projection plane of the virtual camera as a projection point. The projection plane of the virtual camera is usually associated to the virtual focal length of the virtual camera, so the projection plane of the virtual camera can usually be determined according to the virtual focal length of the virtual camera, which is equivalent to obtaining the projection point of each second position on the projection plane according to the virtual focal length. The above-mentioned reference coordinate system can be set anywhere, and it is generally considered to be set on the surgical robot, and preferably, set on the slave operating device. For example, the reference coordinate system is the base coordinate system of the slave operating device. For another example, the reference coordinate system is the tool coordinate system of the robotic arm from the slave operating device.

Further, the realization process of obtaining the projection point of each second position on the projection plane according to the virtual focal length can be divided into the following two steps: the first step is to obtain the contour information of the joint represented (associated) by each feature point, wherein the contour information includes size information and/or line type information, etc.; and the second step is to obtain the projection point of each second position on the projection plane combining the virtual focal length and contour information.

Wherein, the above-mentioned first position, second position and third position may be a point position or an area composed of multiple points. That is, the projection point can be regarded as a point or a point set, which depends on feature points. Further, the feature point itself selects a point in the feature part, the projection point is a point; while the feature point itself selects a point set of the feature part (the concept of "region"), the projected point corresponds to a point set (i.e., region). If the point set of the feature points can reflect the geometric size of the feature part, then the projection point can also reflect the geometric size of the feature part, and then the real structure of the operating arm can be approximately displayed, which is more conducive to displaying the motion state of the operating arm.

In addition, the more or denser the amount of selected feature points, the more the real structure of the operating arm can be displayed approximately. For example, by fitting and connecting these projection point, the linear features of the operating arm, such as straight lines, curves, radians of curves, can be reflected more accurately.

In this step, more specifically, the projection point of each of the plurality of feature points in the feature point sequence of the virtual camera can be determined in combination with the virtual focal length (virtual field of view) and/or virtual aperture (depth of field), the kinematic model and the joint variables of the virtual camera.

As shown in FIG. 8, a projection principle is illustrated. The operating arm includes feature points sequence having feature points Q1, Q2, Q3 and Q4. Under the virtual imaging of the virtual camera, a sequence of projection point is obtained on the projection plane. The sequence of projection point corresponds to q1, q2, and q3 and q4.

Exemplarily, taking the feature points Q1 and Q2 as an example, the positions of Q1 and Q2 in space are obtained according to the kinematic model and joint variables as Q1 (X1, Y1, Z1) and Q2 (X2, Y2, Z2 respectively) ). The projection point q1(x1, y1) and q2(x2, y2) of the feature point Q1 and Q2 on the projection plane are determined in combination with the virtual focal length and can be obtained by the following formula:

$$x1=fx*(X1/Z1)+cx;$$

$$y1=fy*(Y1/Z1)+cy;$$

$$x2=fx*(X12/Z12)+cx;$$

$$y2=fy*(Y12/Z12)+cy;$$

Wherein, fx is the focal length in the horizontal orientation, fy is the focal length in the vertical orientation, cx is the offset relative to the optical axis in the horizontal orientation, and cy is the offset relative to the optical axis in the vertical orientation. Among them, the values of fx and fy can be equal or unequal.

Step S15, fitting and connecting the projection points of each of the plurality of feature points orderly to generate a projected image of the operating arm.

In this step, the projection point can be connected in an orderly manner according to the order of the feature points corresponding to the projection point in the feature point sequence to generate the projected image of the operating arm. The "order" herein refers to a relative order among projection points. It is not about which projection point is connected first and then which projection point is connected later. It is feasible to connect projection points from distal end corresponding to proximal end of the real structure of the operating arm, or from the distal end to the proximal end, or from the middle to the distal end according to the corresponding order between the projection points.

In addition, in this step, each projection point can be fitted and connected in an orderly manner combining the contour information of the feature portion of the operating arm to generate a projected image. For example, when the actual geometric dimensions of each feature portion of the operating arm are approximately the same, each projection point can be connected by a line segment with the same size as the projection point.

In addition, "fit and connect" can refer to the connection method of linear features close to the feature portion, for example, for an operating arm that is generally linear, connecting adjacent projection point with straight line segments; for another example, for at least partially curved operating arm, using a curved segment to connect the projection point corresponding to the curved part. The way of fitting the connection can reflect the linear characteristics of the operating arm.

Continuing to refer to FIG. 8, by orderly fitting and connecting q1, q2, q3 and q4, a projected image corresponding to the operating arm can be obtained.

Step S16, displaying the projected image on the display.

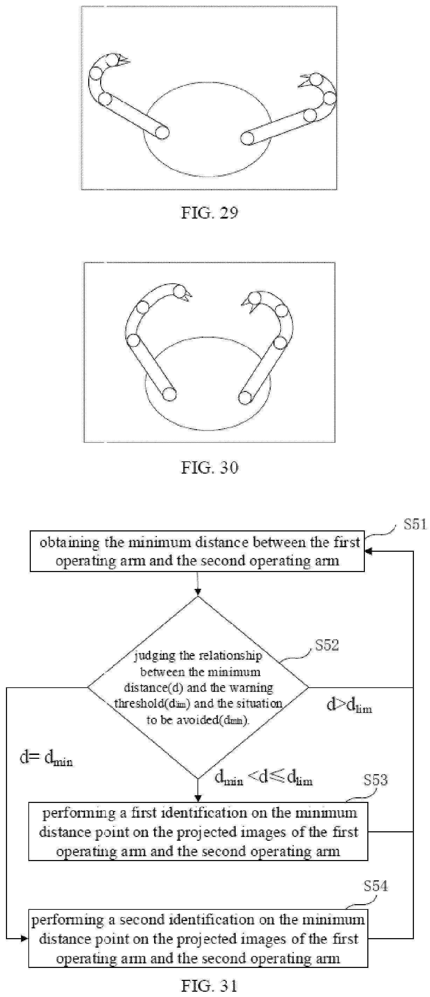

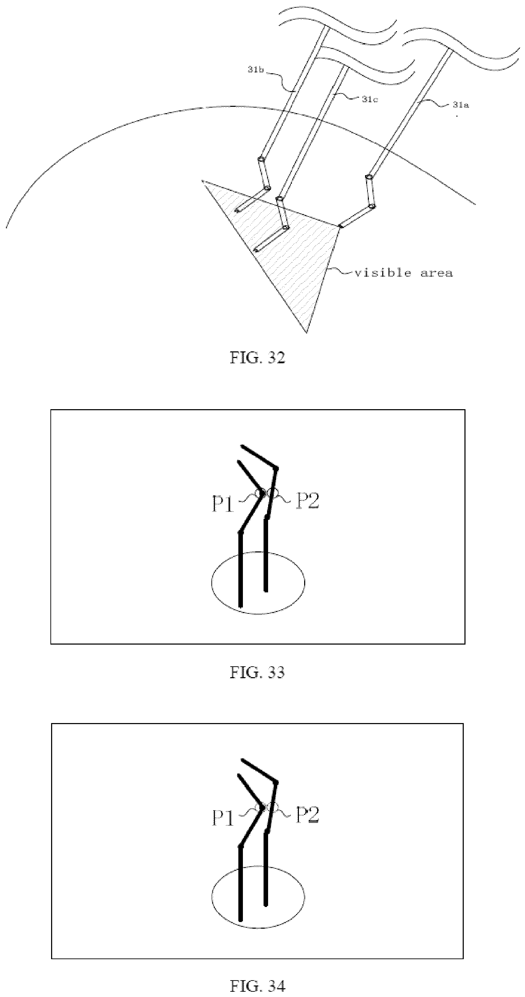

Through the above steps S11 to S16, the doctor can observe the motion state of all operating arms and the complete feature portion of each operating arm through the projected image without a blind spot, which is helpful to assist the doctor to perform operations reliably and continuously. Referring to FIG. 30 and FIG. 33, in FIG. 32, the operating arms 31b and 31c may exist potential collision that cannot be observed outside the real visible area of the real camera. Through the above steps S11 to S16, the potential collision can be observed according to the projected image.

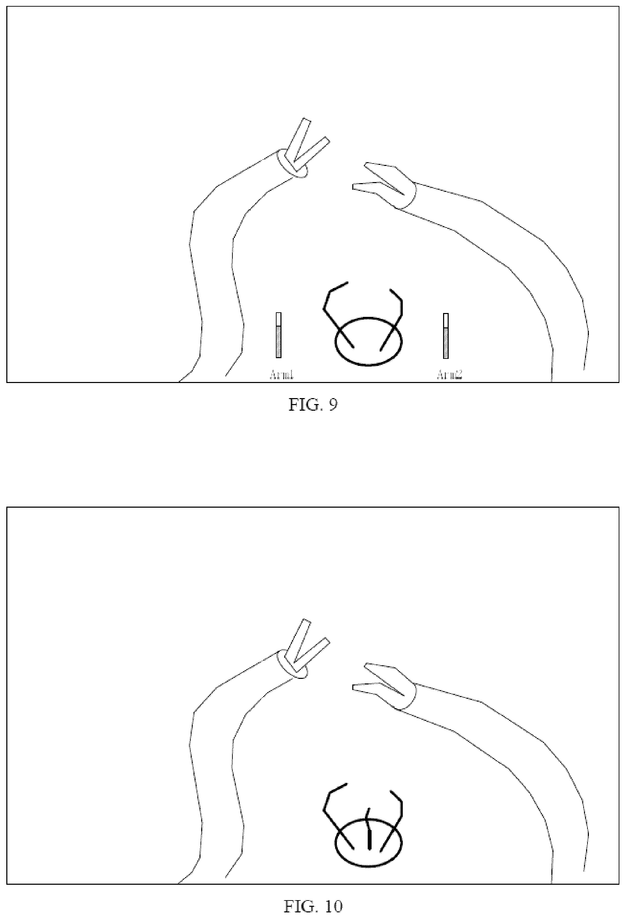

FIG. 9 illustrates a display interface that generates only a projected image of the surgical arm. FIG. 10 illustrates another display interface that simultaneously generates projected images of the surgical arm and the camera arm. The projected images in FIG. 9 and FIG. 10 both reflect the motion state of each feature point corresponding to the operating arm.

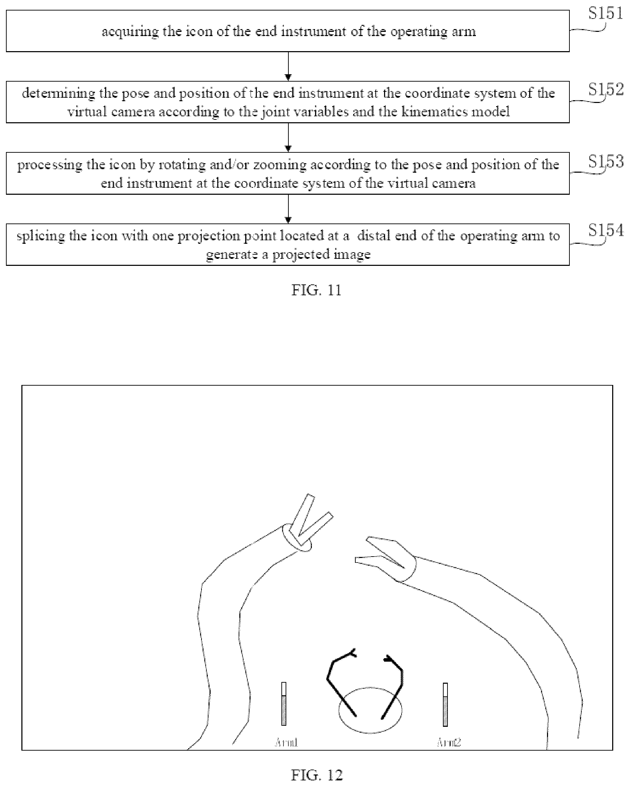

In the above embodiment, for the projected image is generated by a series of projection point connected in an orderly manner, and these feature points may not easily reflect the structural features of the end instrument of the operating arm, in order to more easily reflect the structural features of the end instrument, as shown in FIG. 11, When S15, orderly fitting and connecting each projection point to generate the projected image of the operating arm, the controller can be configured to:

Step S151, acquiring the icon of the end instrument of the operating arm.

For example, obtain the type of the operating arm first, and then match out the icon of the end instrument of the operating arm according to the type of the operating arm. For another example, the icon of the end instrument of the operating arm can be matched according to the acquired feature point sequence. These icons are pre-stored in the storage unit in association with the type of operating arm and/or the sequence of feature points.

Step S152: determine the pose and position of the end instrument in the coordinate system of the virtual camera according to the joint variables and the kinematics model.

Step S153, process the icon by rotating and/or zooming the according to the pose and position of the end instrument in the coordinate system of the virtual camera.

Wherein, the icon is usually zoomed according to the position of the end instrument in the coordinate system of the virtual camera, and the icon is rotated according to the posture (orientation) of the end instrument in the coordinate system of the virtual camera.

Step S154, splicing the icon with one projection point at a distal end of the operating arm to generate a projected image.

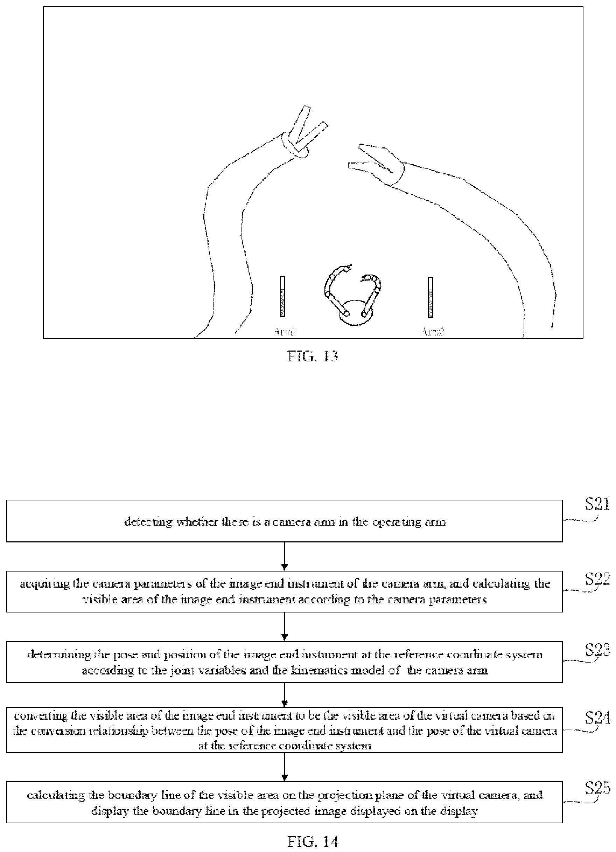

Referring to FIG. 12, a display interface is illustrated, which shows the shape of the end instrument corresponding to operating arm in the projected image, however, the projected image does not reflect the contour shape corresponding to operating arm. Referring to FIG. 13, another display interface is illustrated, which also displays the shape of the end instrument corresponding to operating arm in the projected image, of course, the projected image reflects the contour shape corresponding to operating arm.

In one embodiment, in the surgical robot of the present application, the operating arm includes a camera arm with an image end instrument and/or a surgical arm with an operation end instrument. As shown in FIG. 14, the controller is also configured to the following steps:

Step S21, detecting whether there is a camera arm in the operating arm.

This step S21 can be triggered by the user through the input portion. The detection step can also be implemented by, for example, acquiring the type of the operating arm, and then judging whether the operating arm includes a camera arm according to the type of the operating arm. Of course, all surgery must have a camera arm.

When it is detected in this step that the camera arm is included in the operating arm, the process proceeds to step S22.

In step S22, acquiring the camera parameters of the image end instrument of the camera arm, and calculating the visible area of the image end instrument according to the camera parameters.

Wherein, the camera parameters of the image end instrument include focal length and aperture.

Step S23: determining the pose and position of the image end instrument in a reference coordinate system according to the joint variables and the kinematics model of the camera arm.

Step S24, converting the visible area of the image end instrument to be the visible area of the virtual camera based on the conversion relationship between the pose and position of the image end instrument and the pose and position of the virtual camera in a reference coordinate system.

Step S25, calculating the boundary line of the visible area on the projection plane of the virtual camera, and display the boundary line in the projected image displayed on the display.

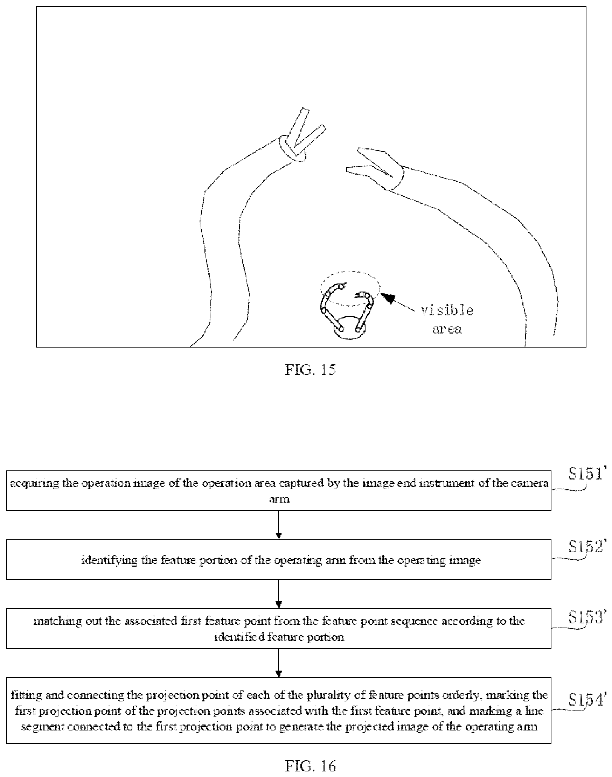

Referring to FIG. 15, a display interface is illustrated, which show the visible area of the image end device in the projected image. The portion outside the visible area is regarded as the non-visible area.

Through the above steps S21 to S25, it is convenient for the doctor to clearly perceive from the projected image which parts of the operating arm are visible and which parts are invisible in the real field of view.

In an embodiment, as shown in FIG. 16, the controller is further configured to the following steps in the above step S15, that is, When orderly fitting and connecting each projection point to generate the projected image of the operating arm:

Step S151', acquiring the operating image of the operation area captured by the image end instrument of the camera arm.

Step S152', identifying the feature portion of the operating arm from the operating image.

Image recognition can be apply. More preferably, image recognition can be performed in combination with a neural network such as a convolutional neural network.

Step S153', matching out the associated first feature point from the feature point sequence according to the identified feature portion.

Wherein, in the feature point sequence, in addition to the first feature points that can be matched, it also includes the second feature points that are not matched. It should be understood that the "first feature point" refers to a type of feature point, and herein it refers to all feature points matched according to the identified feature parts, which may be one or more than two. "Second feature point" refers to another type of feature point, and herein it refers to all remaining feature points in the feature point sequence except the first feature point, which may also be one or more than two.

Step S154', fitting and connecting the projection point of each of the plurality of feature points orderly, marking the first projection point of the projection points associated with the first feature point, and mark a line segment connected to the first projection point to generate the projected image of the operating arm.

It is especially suitable for the case where the feature points are relatively dense, for example, when each feature portion is correspondingly represented by two or more feature points, through the above steps S151' to S154', that is, by marking the first projection point and its connected line segment, It can better show the visible part and invisible part of the operating arm under the end of the image of the operating arm.

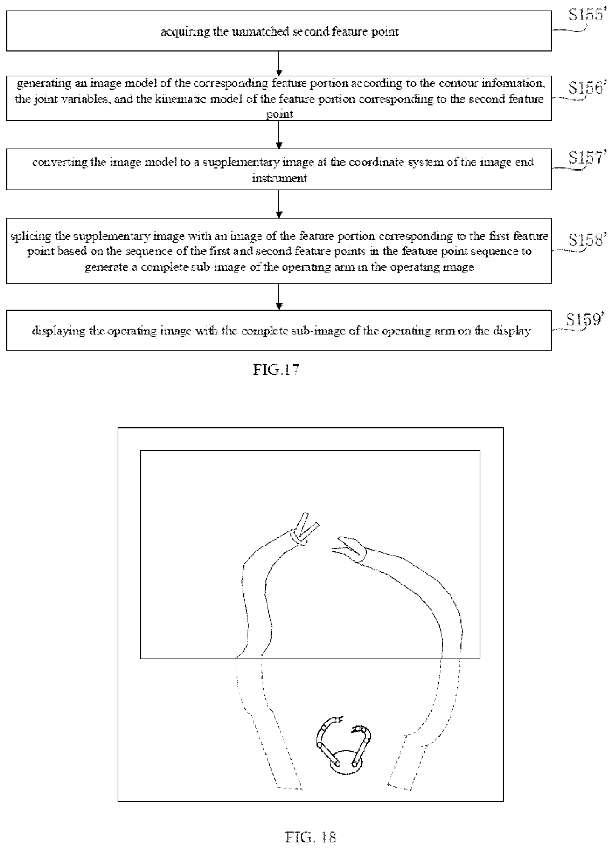

In one embodiment, referring to FIG. 17, the controller may also be configured to perform the following steps in step S153', that is, after matching out the associated first feature point in the feature point sequence according to the identified feature portion:

Step S155', acquiring the unmatched second feature point.

In brief, the second feature point can be obtained by excluding the first feature point in the feature point sequence.

Step S156', generating an image model of the corresponding feature portion according to the contour information, the joint variables and the kinematic model of the feature portion corresponding to the second feature point.

This image model can be a reconstructed computer model or a computationally obtained projected model.

Step S157', converting the image model to a supplementary image in the coordinate system of the image end instrument.

Step S158', splicing the supplementary image with an image of the feature portion corresponding to the first feature point based on the sequence of the first and the second feature points in the feature point sequence to generate a complete sub-image of the operating arm in the operating image.

Step S159', displaying the operating image with the complete sub-image of the operating arm on the display.

FIG. 18 illustrates a display interface supplemented with an operating arm whose operating image is incomplete.

Through the above steps S155' to S159', the doctor can also be assisted in viewing some feature portion of the operating arm that cannot be seen by the real camera.

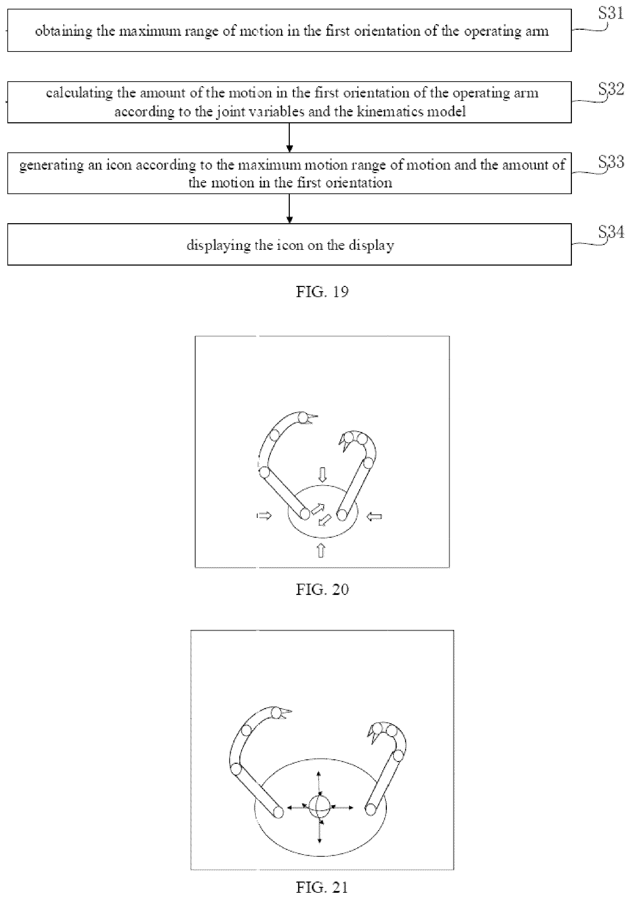

In one embodiment, referring to FIG. 19, the controller may also be configured to perform the following steps:

Step S31, obtaining the maximum range of motion in the first orientation of the operating arm.

Step S32, calculating the amount of the motion in the first orientation of the operating arm according to the joint variables and the kinematics model.

Step S33, generating an icon according to the maximum range of motion and the amount of the motion in the first orientation.

The maximum motion range may be pre-stored in the aforementioned storage unit.

In step S34, displaying the icon on the display.

Such a graphical display can continue to refer to FIG. 9, FIG. 12 and FIG. 13.

The first orientation can be one or more of the forward and backward feed orientation, the left and right motion orientation, the up and down motion orientation, the auto rotation orientation, the pitch orientation, and the yaw orientation, which can be configured according to the effective degrees of freedom of the operating arm. Exemplarily, the first orientation is the forward and backward feed orientation.

The icon can be either a progress bar or a pie, chart. For example, the maximum range of motion is a fixed-length bar in the progress bar, and the amount of motion is a variable-length bar within the length of the fixed-length bar. Wherein, when the motion amount increases or decreases, the color of the variable length bar can be darkened or lightened accordingly. In addition, the proportional value of the exercise amount in the maximum motion range can also be calculated separately or in combination and displayed in the display area of the progress bar, for example, displayed in a variable-length bar of the motion amount.

Through the above steps S31 to S34, it can play a role in mentioning a doctor on the range of motion in the corresponding orientation.

In one embodiment, the controller may be further configured to detect the currently controlled first operating arm from the operating arm, thereby identifying the first operating arm in the projected image. In this way, the controlled and uncontrolled operating arms can be displayed differentially in the display. Wherein, whether the operating arm is controlled can be determined according to whether a start command for actively controlling the operating arm is detected.

In the above embodiment, different virtual cameras that can be selected by the input portion have different pose and positions in a reference coordinate system, so as to simulate a real camera such as an image end instrument to observe the operating arm from different positions and/or poses (orientation).

In one embodiment, the pose and position of the virtual camera in a reference coordinate system may be determined based on the reachable workspace (referred to as the reachable space) of the operating arm in a reference coordinate system. This allows the pose and position of the virtual camera to be associated to its reachable workspace for easy determination.

Further, the pose and position of the virtual camera in a reference coordinate system can be determined based on the union space of the reachable workspace of the operating arm in a reference coordinate system. When there is only one operating arm, this union space is equal to the reachable workspace of the operating arm. When there are two or more operating arms, this union space is the space corresponding to the union of the reachable workspace of the operating arms. Wherein, the reachable workspace of each operating arm in a reference coordinate system can be determined according to the kinematic model of the operating arm, and stored in the aforementioned storage unit for direct recall. Of course, the reachable workspace of each operating arm in a reference coordinate system can also be recalculated one or more times each time the surgical robot is activated according to the kinematic model of the operating arm.

Furthermore, the position of the virtual camera in a reference coordinate system is always located outside the union space, and the posture of the virtual camera in a reference coordinate system is always oriented toward the union space.

Such determined pose and position of the virtual camera can always fully observe the motion state of each operating arm, including observing the motion state of each operating arm and observing the motion state among the operating arms.

The virtual camera is configured with selectable virtual focal length. In one embodiment, the position of the virtual camera only needs to be located outside the area determined by the shortest virtual focal length that can see the entire union space. In one embodiment, the position of the virtual camera is also feasible as long as the position of the virtual camera is located within the area determined by the minimum virtual focal length that can see the entire union space. In one embodiment, the position of the virtual camera may be jointly defined by the longest focal length and the shortest focal length available for configuration, and it is located at the intersection between the first area determined by the longest focal length and the second area determined by the shortest focal length area.

The pose and position (orientation) of the virtual camera is always towards a relatively certain point or area in the union space. In one embodiment, the pose and position of the virtual camera is always toward the center of the union space. In this way, it can be ensured that the virtual imaging plane of the virtual camera can always perform virtual imaging of each operating arm.

In one embodiment, the controller may be configured to display the projected image in a first display window of the display, and to generate an icon of a selectable virtual camera in the first display window.

Wherein, the relative position of the icon corresponding to the virtual camera and the projected image may be fixed and synchronously transformed with the transformation of the viewpoint of the projected image. The transformation of the projected image viewpoint (i.e., coordinate) is related to the position of the selected virtual camera.

Exemplarily, the icons corresponding to the virtual cameras may be set to six, that is, six different positions representing virtual cameras. Wherein proceed virtual imaging so as to generate a projected image under the corresponding viewpoint from left, right, up, bottom, front and rear, respectively.

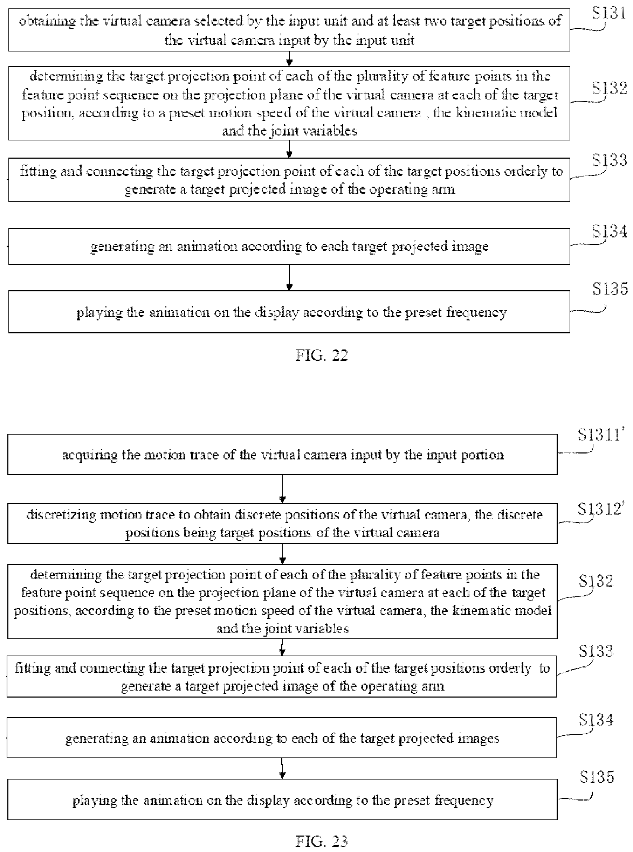

Exemplarily, the icons can be appear as either an arrow or a camera, and any one of the icons rotated and selected corresponds to a virtual camera. The icon can also be, for example, a dot, a circle, or the like. As shown in FIG. 22, the icon is displayed as an arrow pattern, and the arrow shows the adjustment orientation of the field angle.

Exemplarily, the icon can be appeared as a rotatable sphere, and any position reached by the sphere that is rotated corresponds to a virtual camera. For example, any position on the surface of the sphere may correspond to some positions of the aforementioned first area, the second area and/or the intersection area of the first area and the second area, so any position where the sphere is rotated can represent a virtual camera. Of course, the postures of these virtual cameras are all directed to a certain point in the reachable space, so as to ensure that each complete operating arm can be seen. As shown in Figure 23, the icon is displayed as a sphere, and the arrow shows the adjustable orientation of the field angle.

In one embodiment, referring to FIG. 22, in the above-mentioned step S13 of acquiring the virtual camera selected by the input portion, the controller is configured to:

Step S131, acquiring the virtual camera selected by the input portion and at least two target positions of the virtual camera input by the input portion.

In this step, the selected virtual camera mainly refers to the virtual focal length and/or virtual aperture of the selected virtual camera; the input at least two target positions of the virtual camera may be two or more discrete positions, or two or more continuous positions.

When inputting the target position, a tracking mode can be set at the same time, such as single tracking projection mode, multiple tracking projection mode and reciprocating tracking projection mode. Some columns of target positions include the start position A and the end position B. For the single-tracking projection mode, only one projection of each target position in A to B is performed; for the multiple-tracking projection mode, The projection of each target position in A to B is performed a specified number of times; for the reciprocating tracking projection mode, the projection of each target position in A to B is repeated. For the single-track projection mode and the multiple-track projection mode, after the entire projection process from A to B is completed, the virtual camera can stay at a specified position to continuously project, and the specified position can be any of A to B. A position, such as A or B, can also be other default positions.

Step S132, determining the target projection point of each of the plurality of feature points in the feature point sequence on the projection plane of the virtual camera at each of the target position, according to the preset motion speed of the virtual camera , the kinematic model and the joint variables.

Step S133, fitting and connecting the target projection point of each of the target position orderly to generate a target projected image of the operating arm.

Step S134, generating an animation according to each target projected image.

Step S135, playing the animation on the display according to the preset frequency.

Through the above steps S131 to S135, the doctor can dynamically observe the mutual positional relationship and projection information of the operating arms, so as to solve the situation of partial information overlap or projection distortion under a single viewing angle, and to understand the spatial position information from multiple directions.

In one embodiment, in the above stepS13 of acquiring the virtual camera selected by the input portion: FIG. 23, the controller is configured to:

Step S1311', acquiring the motion trace of the virtual camera input by the input portion.

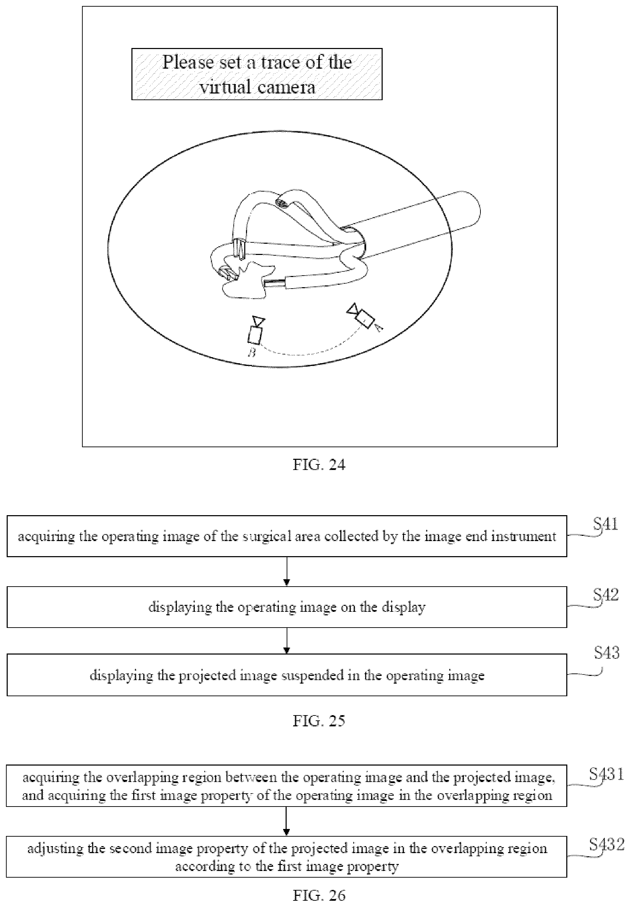

For example, the motion trace may be a track of cursor movement, and for example, the motion trace may be a sliding track of a finger. For ease of implementation, exemplarily, the starting position of the motion trace is the position of the virtual camera corresponding to one of the aforementioned icons, and the starting position has coordinates (x0, y0, z0), in the motion trace, other positions of coordinates of the Z-axis remain unchanged, but only the X-axis and Y-axis coordinates are changed. In other embodiments, the starting position of the motion trajectory is not necessarily the position of the virtual camera corresponding to one of the aforementioned icons, but it is usually necessary to first specify the Z-axis coordinates of the entire trajectory, and then only change the X-axis and Y-axis coordinates. As shown in FIG. 24, it illustrates a configuration interface of a virtual camera motion trajectory.

Step S1312', discretizing motion trace to obtain discrete positions of the virtual camera, the discrete positions being target positions of the virtual camera.

Step S132, determining the target projection point of each of the plurality feature points in the feature point sequence on the projection plane of the virtual camera at each of the target positions, according to the preset motion speed of the virtual camera, the kinematic model and the joint variables.

Step S133, fitting and connecting the target projection point of each of the target positions orderly to generate a target projected image of the operating arm.

Step S134, generating an animation according to each of the target projected images.

Step S135, playing the animation on the display according to the preset frequency.

In one embodiment, as shown in FIG. 25, in the following steps, the controller is also generally configured to:

Step S41, acquiring the operating image of the surgical area collected by the image end instrument.
Step S42, displaying the operating image on the display.
Step S43, displaying the projected image in suspension in the operating image.

This means that the position of the projected image in the operating image can be changed easily. For example, the display having a floating window displaying the projected image, and the remaining area of the display displays the operating image, which helps to allow the projected image to avoid some key positions in the operating image as needed to facilitate the operation.

In one embodiment, as shown in FIG. 26, in the step S43 of displaying the projected image suspended in the operating image, the controller may also be configured to:

Step S431, acquiring the overlapping region between the operating image and the projected image, and acquiring the first image property of operating image in the overlapping region.

Step S432, adjusting the second image property of the projected image in the overlapping region according to the first image property.

These image properties include a combination of one or more than two among color, saturation, hue, brightness, and contrast. For example, a combination of one or more than two among color, brightness, and contrast.

Through the above steps S431 to S432, the image property of the projected image can be adjusted adaptively according to the image attributes of the operating image. For example, when the operating image is dark, the projected image can be brightened, or the color of the projected image can be changed to make the projected image more prominent relative to the operating image for easy observation by the doctor.

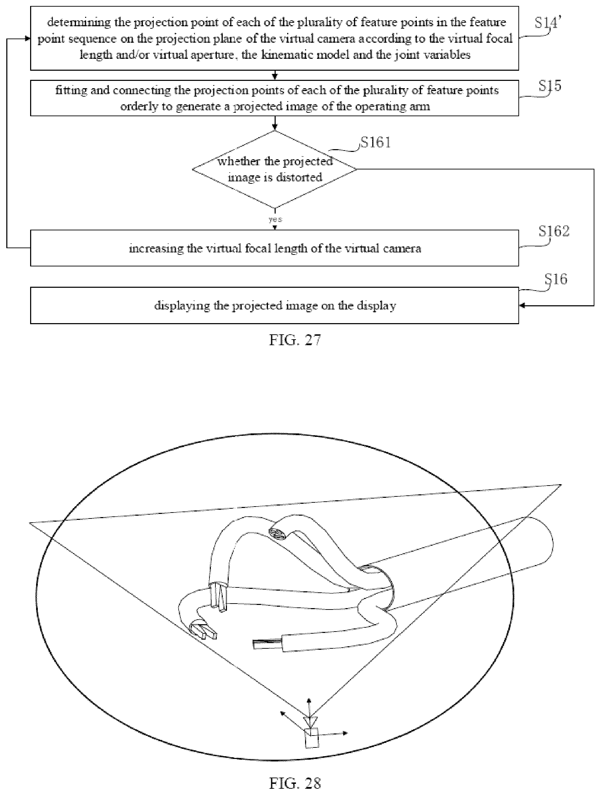

According to the invention, as shown in FIG. 27, before the step S16 of displaying the projected image on the display, the controller is also configured to:

Step S161, detecting whether the projected image is distorted.

When it is detected that the projected image is distorted, proceed to step S162; and when it is detected that the projected image is not distorted, proceed to step S16.

Exemplarily, whether the projected image is distorted can be judged as follows: Step 1, obtain the position of each projection point in a reference coordinate system; Step 2, obtain the amount of the first projection point that fall within the edge area of the projection points; Step 3, calculate the ratio of the amount of the first projection point to the total number of projection point, and when the ratio reaches a threshold, it is determined that the projected image is distorted.

The edge area can be obtained based on a display window or a projection plane in which the projected image is displayed.

Step S162, increasing the virtual focal length of the virtual camera.

That is, reducing the field angle according to the almost inverse relationship between the focal length and the field angle.

Step S14', determining the projection point of each of the plurality of feature points in the feature point sequence on the projection plane of the virtual camera according to the virtual focal length and/or virtual aperture, the kinematic model and the joint variables.

Referring to FIGS. 28 to 30, wherein FIG. 28 shows a schematic diagram of observing the operating arm with a large field angle; and FIG. 29 shows the display interface having a first projected image generated under the field angle shown in FIG. 28. It can be seen that the edge area of the projected image is distorted if there is a compression problem; FIG. 30 illustrates the re-generated display interface having the second projected image after the FOV is adjusted. It can be seen that the edge area of the projected image is expanded, which eliminates the distortion problem.

The foregoing step S162 may be exemplarily increasing the virtual focal length of the virtual camera by a proportional factor. In short, it can be re-determined according to formula (1): F=k*f, k is the adjustment coefficient, k>1; f is the focal length before adjusting; F is the adjusted focal length.

In one embodiment, the virtual focal length can also be re-determined according to the following formula (2).

$$fx=k1*Fx*fx0;$$

Wherein, fx0 is the focal length at the center of the projection plane; Fx is the distance from the center of a projection point on the projection screen along the X-axis orientation; k1 is the setting coefficient; fx is the focal length in the x-orientation of a certain projection point. In order to increase the virtual focal length, it is only necessary to satisfy k1*Fx>1.

The formula (2) associates the virtual focal length of the virtual camera with the position of the projection point, that is, the virtual focal length is related to the position of the projection point, and the virtual focal length to be adjusted changes with the change of the projection point. Among them, x represents any point in the projection plane, and the position of the projection point P in the projection plane is represented as P(Fx, Fy).

According to the same principle of formula (2), it is also possible to determine the virtual focal length in the vertical orientation, re-determine the relative optical axis offset cx in the horizontal orientation, and re-determine the relative optical axis offset cx in the vertical orientation, which can be achieved by the following similar methods respectively:

$$fy=k2*Fy*fy0;$$

$$cx=k3*Fx*cx0;$$

$$cy=k4*Fy*cy0;$$

Through the above steps S161 to S162, by expanding the projected image, the problem that losing the validity of information observation caused by the feature point of the field angle having projection compression under the condition of a large field angle can be solved.

In one embodiment, the controller may also be configured to:

The operating instruction for displaying the image of the corresponding operating arm is obtained, then the image of the corresponding operating arm is displayed or hidden according to the operating instruction.

Specifically, when the operation instruction for the display image of the operating arm is acquired, the projection point corresponding to the operating arm is determined in step S14. However, when the operation instruction for the hidden image of the operating arm is obtained, it is correspondingly unnecessary to determine the projection point corresponding to the operating arm in step S14. This is equivalent to a customized configuration of the projected image, so as to simplify the projected image and remove interfering sub-images. In one embodiment, a similar purpose can be achieved at least in part by adjusting the virtual aperture (virtual depth of field) of the virtual camera. For example, the operating arm far away from the virtual camera can be blurred by adjusting the virtual aperture, so that only clear Virtual imaging of the operating arm adjacent to the virtual camera.

In an embodiment, the above-mentioned graphical display method may further include that when the first operating arm reaches the threshold of the event, at least a portion of the first operating arm is identified in the projected image and displayed on the display.

Wherein, the first operating arm also refers to a type but not limited to a specific operating arm. The threshold is a warning threshold and the event is a situation to be avoided.

In a specific embodiment, the warning threshold is based on the distance between the first operating arm and the second of the operating arms, for example, the warning threshold may be a numerical value. The situation to be avoided is a collision between the first operating arm and the second operating arm, for example, the situation to be avoided may be a numerical value. The second operating arm also refers to a type but not limited to a specific of the operating arms. For example, as shown in Figure 31, the method can be implemented by the following steps:

Step S51, obtaining the minimum distance between the first operating arm and the second operating arm.

This step S51 is proceeded in real time.

Step S52, judging the relationship between the minimum distance and the warning threshold and the situation to be avoided.

The warning threshold and the situation to be avoided are represented by numerical values, and the situation to be avoided is a collision between the first operating arm and the second operating arm, the numerical value $d_{lim}$ represented by the warning threshold is greater than that the value $d_{min}$ represented by the situation to be avoided, that is, $d_{lim} > d_{min}$, the minimum distance between the first operating arm and the second operating arm is represented by d. In one embodiment, if $d_{min} = 0$, it represents a collision.

In step S52, if $d > d_{lim}$, that is, the minimum distance does not reach the warning threshold, proceed to step S51; if $d_{min} < d \leq d_{lim}$, that is, the minimum distance reaches the warning threshold and does not reach the situation to be avoided, proceeds to step S53; if $d = d_{min}$, that is, the minimum distance exceeds the warning threshold and reaches the situation to be avoided, proceeds to step S54.

Step S53, performing a first identification on the minimum distance point on the projected images of the first operating arm and the second operating arm.

As shown in FIG. 32, the operating arm includes the camera arm 31a, the surgical arm 31b and the surgical arm 31c, and the minimum distance between the surgical arm 31b and the surgical arm 31c reaches the warning threshold. At this time, in step S53, a color or a graphic, such as circles, can be used as a mark to the minimum distance points P1 and P2 in the projected images of the surgical arm 31b (i.e., the first operating arm) and the surgical arm 31c (i.e., the second operating arm), as shown in FIG. 33. When it is re-detected that the minimum distance does not reach the warning threshold, generally, the identification of the minimum distance point on the projected images of the first operating arm and the second operating arm may be eliminated. When it is re-detected that the minimum distance reaches the situation to be avoided, proceeds to step S54, that is, the second identification is performed.

In addition, in the process of performing the first identification, that is, when the condition of $d_{min} < d \leq d_{lim}$ is satisfied, the first identification may be changed as the minimum distance gradually decreases or increases. For example, the color is gradually transformed, but it can be different from the color when $d = d_{min}$; for example, the first logo is stroboscopic, but it can be different from the stroboscopic when $d = d_{min}$.

Step S54, performing a second identification on the minimum distance point on the projected images of the first operating arm and the second operating arm.

The first identification is different from the second identification. In step S54, for example, the identification of the minimum distance points P1 and P2 in the models of the first operating arm and the second operating arm can be enhanced, such as by deepening the color; or, the identification of the minimum distance points of the first operating arm and the second operating arm can be flickered; or the identification of the minimum distance point of the first operating arm and the second operating arm can be changed, such as by changing the type of graphic; as shown in FIG. 34, the solid line circles shown in FIG. 33 are replaced by dashed line circles in FIG. 34. When it is re-detected that the minimum distance reaches the warning threshold and the situation to be avoided is not reached, proceeds to step S53, that is, performing the first identification.

Steps S51 to S54 help the doctor to grasp the collision position between the operating arms.

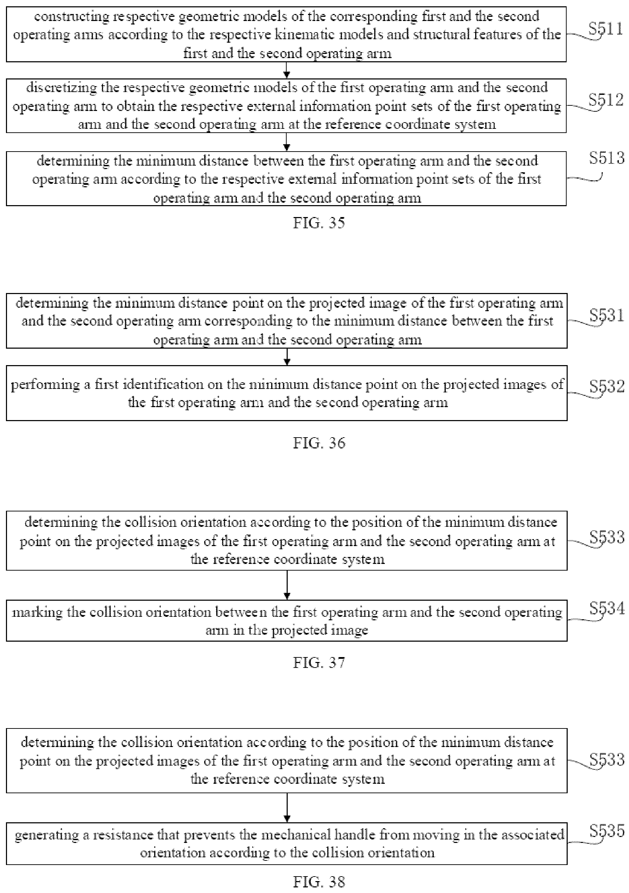

More specifically, as shown in FIG. 35, the above step S51 can be implemented by the following steps:

Step S511, constructing respective geometric models of the corresponding first and second operating arms according to the respective kinematic models and the structural features of the first and the second operating arm.

In step S511, instead of an actual model, a slightly larger basic geometry can usually be applied to perform the interference analysis, so as to improve the detection efficiency. The respective geometric models of the first operating arm and the second operating arm can be simplified into, for example, a sphere, a cylinder, a cuboid, a convex polyhedron, or a combination of two or more.

Step S512, discretizing the respective geometric models of the first operating arm and the second operating arm to obtain the respective external information point sets of the first operating arm and the second operating arm in a reference coordinate system.

In step S512, digitizing the respective geometric models of the first operating arm and the second operating arm to obtain their respective external information point sets.

Step S513, determining the minimum distance between the first operating arm and the second operating arm according to the respective external information point sets of the first operating arm and the second operating arm.

In step S513, a minimum distance can be determined by applying the distance tracking method. More specifically, the minimum distance can be determined by applying a traversal on the set of external information points of the first operating arm and the second operating arm.

More specifically, as shown in FIG. 36, the above step S53 can be implemented by the following steps:

Step S531, determining the minimum distance point on the projected image of the first operating arm and the second operating arm corresponding to the minimum distance between the first operating arm and the second operating arm.

Step S532, performing a first identification on the minimum distance point on the projected images of the first operating arm and the second operating arm.

In one embodiment, as shown in FIG. 37, when the minimum distance reaches the warning threshold, the graphical display method may further includes the following steps:

Step S533, determining the direction of collision according to the position of the minimum distance point on the projected images of the first operating arm and the second operating arm in a reference coordinate system.

Step S534, marking the direction of collision between the first operating arm and the second operating arm in the projected image.

The above-mentioned identification of the minimum distance point and the direction of collision between the first operating arm and the second operating arm in the projected image, such as marking the direction of collision by an arrow, can provide visual feedback for the doctor to avoid collision.

The handle of the main console adopts a mechanical handle. In an embodiment, as shown in FIG. 38, corresponding to the situation in the above-mentioned step S53, that is, when the minimum distance reaches the warning threshold and does not reach the situation to be avoided, it includes:

Step S533, determining the direction of collision according to the position of the minimum distance point on the projected images of the first operating arm and the second operating arm in a reference coordinate system.

Step S535, generating a resistance that prevents the mechanical handle from moving in the associated orientation according to the direction of collision.

This provides force feedback to the doctor to avoid collisions when there is a tendency to collide between the operating arm arms.

Specifically, the mechanical handle includes a plurality of joint components, a sensor sensed the joint coupled to the controller, and a driving motor coupled to the controller for driving each joint component. More specifically, generating a resistance against the movement of the mechanical handle in the associated orientation according to the direction of collision can be illustrated as: to generate a reverse torque in the associated orientation according to the resistance.

When the minimum distance is between the warning threshold and the situation to be avoided, for example, the magnitude of reverse torque can be constant; for another example, the magnitude of the reverse torque is negatively correlated with the magnitude of the minimum distance. When the magnitude of the reverse torque is negatively correlated with the magnitude of the minimum distance, specifically, when the minimum distance gradually decreases, the reverse torque is increased to generate greater resistance; and when the minimum distance gradually increases, the reverse torque decreases. For example, the change of the reverse torque may be linear; for example, the change of the reverse torque may be non-linear such as stepwise. When the minimum distance reaches the situation to be avoided, the generated reverse torque can be at least the smallest magnitude to completely obstruct the motion of the mechanical handle in the direction of collision. In one embodiment, the force sensor provided by each joint component of the mechanical arm handle can detect the force or torque applied by doctor, which in turn generating a reverse torque based on the force or torque applied by the doctor that at least counteracts the force applied by the doctor. In one embodiment, a force that is large enough can be generated suddenly, so that a doctor cannot move the mechanical handle in the direction of collision with ordinary strength.

In one embodiment, the warning threshold may also be based on the range of motion of at least one joint assembly in the first operating arm, and the situation to be avoided is the limitation of the range of motion of at least one joint assembly in the first operating arm. Likewise, when the first operating arm reaches the warning threshold, at least the related joint assembly of the model of the first operating arm may be identified in the first display window or the second display window. Furthermore, resistance to motion of the first operating arm over the warning threshold towards the situation to be avoided may also be created at the mechanical handle. This resistance is also achieved by the reverse torque generated by the associated drive motor.

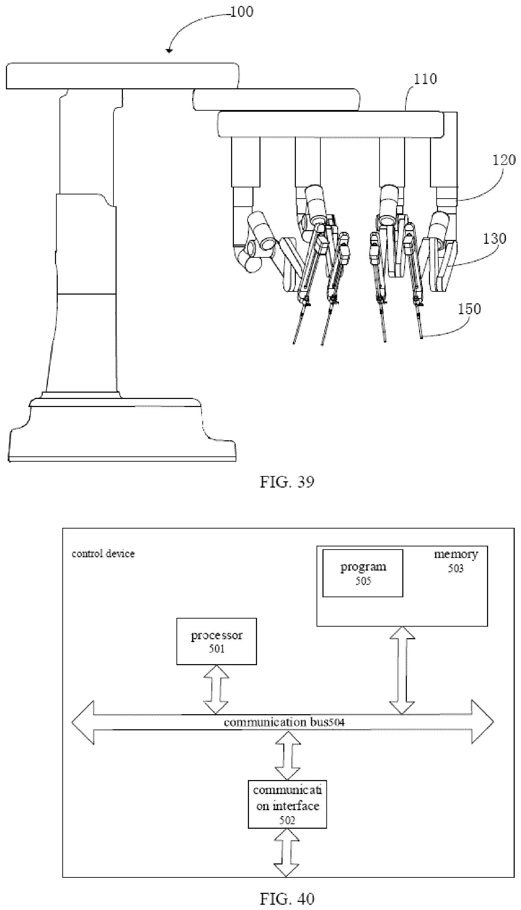

The surgical robot of the above-described embodiment may also be a multiple-port surgical robot. The difference between the multi-port surgical robot and the single-port surgical robot is mainly in the operating equipment. Figure 39 illustrates a slave operating device of a multi-port surgical robot. The robotic arm of the slave operating device in the multi-port surgical robot has a main arm 110, an adjustment arm 120 and an operating arm 130 which are connected in sequence. There are more than two adjustment arms 120 and operating arm 130, such as four. The distal end of the main arm 110 has a directional platform, the proximal ends of the adjustment arms 120 are connected to the directional platform, and the proximal end of the operating arm 130 is connected to the distal end of the adjustment arms 120. The operating arm 130 is used for detachably connecting the operating arm 150, and the operating arm 130 has a plurality of joint components. In the multiple-port surgical robot, different operating arms 150 are inserted into the patient through different trocars. Compared with the operating arm 31 of the single-port surgical robot, the operating arm 150 of the multi-port surgical robot generally has less degrees of freedom. Generally, the operating arm 150 only has the degree of freedom of attitude (ie, the degree of freedom of orientation), of course, the change of its attitude generally also affects the position, but because the effect is small, it can usually be ignored. The position of the operating arm 150 is often achieved by the operating arm 130. Since the operating arm 130 and the operating arm 150 are linked to realize the pose and position change, they can be regarded as an operating arm component, which are equivalent to the operating arm 31 in the single-port surgical robot.

In some embodiments, as shown in FIG. 40, the graphical control device may include: a processor 501, a communication interface 502, a memory 503, and a communication bus 504.

The processor 501, the communication interface 502, and the memory 503 communicate with each other through the communication bus 504.

The communication interface 502 is used to communicate with network elements of other devices such as various types of sensors or motors or solenoid valves or other clients or servers.

The processor 501 is configured to execute the program 505, and specifically may execute the relevant steps in the foregoing method embodiments.

Specifically, the program 505 may include program code, which includes computer operation instructions.

The processor 505 may be a central processing unit (CPU), or an application specific integrated circuit (ASIC), or one or more integrated circuits configured to implement the embodiments of the present application, or a graphics processing unit (GPU). One or more processors included in the control device may be the same type of processors, such as one or more CPUs, or one or more GPUs; or may be different types of processors, such as one or more CPUs and one or more GPUs.

The memory 503 is used to store the program 505. The memory 503 may include high-speed RAM memory, and may also include non-volatile memory, such as at least one disk memory.

The program 505 can be specifically used to make the processor 501 performing the following operations: obtain the feature point sequence of the operating arm and its corresponding kinematic model; obtain the joint variables sensed by the sensor, and obtain the virtual camera selected by the input part; determine the projection point of each of the plurality of feature points in the feature point sequence on the projection plane of the virtual camera according to the kinematic model and the joint variables; orderly fit and connect the projection point to generate the projected image of the operating arm; and display the projected image on the display.

The various technical features of the above-described embodiments may be combined in any combination, so that the description is concise, and all possible combinations of the various technical features in the above-described embodiments are described. However, as long as the combination of these technical features does not conflict, it is to be understood that the scope of the present specification is not to be taken in a limiting sense.

**Claims**

1. A surgical robot, comprising:

an input portion;

a display (22);

an operating arm (31) comprising a plurality of joints and a plurality of sensors, the plurality of sensors being configured to sense joint variables of the plurality of joints, the operating arm (31) further comprising a feature point sequence composed of a plurality of feature points, the plurality of feature points being arranged orderly, and each of the plurality of joints being associated with at least one of the plurality of feature points; and

a controller, coupled to each of the input portion, the display (22), and the plurality of sensors, the controller being configured to:

> obtain the feature point sequence of the operating arm (31) and a kinematic model corresponding to the feature point sequence;
>
> obtain the joint variables sensed by the plurality of sensors, and obtain a virtual camera selected by the input portion, the virtual camera comprises at least one of a virtual focal length and a virtual aperture;
>
> determine a projection point of each of the plurality of feature points in the feature point sequence on a projection plane of the virtual camera according to the kinematic model, the joint variables, and the at least one of the virtual focal length and the virtual aperture;
>
> fit and connect the projection point of each of the plurality of feature points orderly to generate a projected image of the operating arm (31);
>
> detect whether the projected image is distorted;
>
> when the projected image distortion is detected to be distorted, increase the virtual focal length of the virtual camera, and repeat a process of determining the projection point of each of the plurality of feature points in the feature point sequence on the projection plane of the virtual camera according to the kinematic model, the joint variables, and the at least one of the virtual focal length and the virtual aperture; and
>
> display the projected image on the display (22) when the image is detected to be not distorted.

2. The surgical robot of claim 1, **characterized in that**, when determining the projection point of each of the plurality of feature points in the feature point sequence on the projection plane of the virtual camera according to the kinematic model and the joint variables, the controller is further configured to:

acquire a first position of each of the plurality of feature points of the feature point sequence in a reference coordinate system based on the kinematic model and the joint variables;

convert the first position of each of the plurality of feature points to a second position in a coordinate system of the virtual camera;

acquire a virtual focal length of the virtual camera and determine the projection plane of the virtual camera based on the virtual focal length; and

acquire a projection point of the second position of each of the plurality of feature points on the projection plane based on the virtual focal length.

3. The surgical robot of claim 1, **characterized in that**, when determining the projection point of each of the plurality of feature points in the feature point sequence on the projection plane of the virtual camera according to the kinematic model and the joint variables, the controller is further configured to:

> acquire a first position of each of the plurality of feature points in a reference coordinate system based on the kinematic model and the joint variables;
>
> convert the first position of each of the plurality of feature points to a second position in a coordinate system of the virtual camera;
>
> acquire a contour information of each of the plurality of joints corresponding to the corresponding feature points which is associated with the joint;
>
> acquire a projection point of the second position of each of the plurality of feature points on the projection plane according to the virtual focal length and the contour information; and
>
> when fitting and connecting the projection point of each of the plurality of feature points orderly to generate the projected image of the operating arm (31), the controller is further configured to: fit and connect the projection point of each of the plurality of feature points orderly to generate the projected image of the operating arm (31) according to the contour information and an order of the plurality of feature points corresponding to the projection points of the plurality of feature points in the feature point sequence.

4. The surgical robot of claim 1, **characterized in that**, when fitting and connecting the projection point of each of the plurality of feature points orderly to generate the projected image of the operating arm (31), the controller is further configured to:

> acquire a type of the operating arm (31), and match out an icon of an end instrument (34) of the operating arm (31) according to the type;
determine a pose and position of the end instrument (34) on the projection plane of the virtual camera based on the joint variables and the kinematic model;
process the icon by rotating and/or zooming based on the pose and position of the end instrument (34) on the projection plane of the virtual camera; and
splice the icon with one projection point located at a distal end of the operating arm (31) to generate the projected image.

5. The surgical robot of claim 1,

  wherein when detecting whether the projected image is distorted, the controller is configured to:

    acquire a position of the projection point of each of the plurality of feature points in a reference coordinate system;
    acquire a quantity of first projection points, wherein the first projection points are the projection points which fall within an edge area of the projection plane or an edge area of a display window, the display window being configured for displaying the projected image on the display (22); and

    calculate a ratio of the quantity of the first projection points to a total quantity of the projection points, and determine that the image is distorted when the ratio reaches a threshold.

6. The surgical robot of claim 1, **characterized in that**,

  the operating arm (31) comprises a camera arm (31a) with an image end instrument (34A), and the controller is further configured to:

    acquire a camera parameter of the image end instrument (34A) of the camera arm (31a), and calculate a visible area of the image end instrument (34A) according to the camera parameter, the camera parameter comprising a focal length and an aperture;
    determine a pose and position of the image end instrument (34A) in a reference coordinate system based on the joint variables and the kinematic model of the camera arm (31a);
    convert the visible area of the image end instrument (34A) to a visible area of the virtual camera based on a conversion relationship between the pose and position of the image end instrument (34A) and the pose and position of the virtual camera in the reference coordinate system; and
calculate a boundary line of the visible area of the virtual camera on the projection plane, and display the boundary line in the projected image on the display (22).

7. The surgical robot of claim 1, **characterized in that**,

  the operating arm (31) comprises a camera arm (31a) with an image end instrument (34A) and a surgical arm (31c) with an operating end instrument (34B);
  when fitting and connecting the projection point of each of the plurality of feature points orderly to generate the projected image of the operating arm (31), the controller is further configured to:

    acquire an operating image of a surgical area captured by the image end instrument (34A) of the camera arm (31a);
    identify a feature portion of the surgical arm (31c) from the operating image;
    match out an associated first feature point from the feature point sequence according to the identified feature portion; and
    fit and connect the projection point of each of the plurality of feature points orderly, mark a first projection point from the projection points associated with the first feature point, and mark a line segment connected to the first projection point to generate the projected image of the operating arm (31);

  wherein the feature point sequence further comprises an unmatched second feature point,
  after matching out the associated first feature point from the feature point sequences based on the identified feature portion, the controller is further configured to:

    acquire the unmatched second feature point;
    generate an image model of the feature portion according to the contour information, the joint variables, and the kinematic model of the feature portion corresponding to the second feature point;
    convert the image model to a supplementary image in a coordinate system of the image end instrument (34A);
    splice the supplementary image with an image of the feature portion corresponding to the first feature point based on the sequence of the first and the second feature points in the feature point sequence to generate a complete sub-image of the operating arm (31) in the operating image; and

display the operating image with the complete sub-image of the operating arm (31) on the display (22).

8. The surgical robot of claim 1, **characterized in that**, the controller is further configured to:

acquire a maximum range of motion in a first orientation of the operating arm (31);
calculate an amount of the motion in the first orientation of the operating arm (31) based on the joint variables and the kinematic model;
generate an icon based on the maximum range of motion and the amount of the motion in the first orientation; and
display the icon on the display (22).

9. The surgical robot of claim 1, **characterized in that**,

a plurality of virtual cameras selectable by the input portion have difference poses and positions in a reference coordinate system; and
the poses and positions of the plurality of virtual cameras in the reference coordinate system are determined by a reachable workspace of the operating arm (31) in the reference coordinate system;
the controller is further configured to display the projected image on a first display window of the display (22), and to generate a plurality of icons of the plurality of virtual cameras.

10. The surgical robot of claim 1, **characterized in that**, when obtaining the virtual camera selected by the input portion, the controller is further configured to:

obtain the virtual camera selected by the input portion and at least two target positions of the virtual camera input by the input portion;
determine a target projection point of each of the plurality of feature points in the feature point sequence on a projection plane of the virtual camera at each of the target positions, according to a preset speed of the virtual camera, the kinematic model, and the joint variables;
fit and connect the target projection point of each of the target positions orderly to generate a target projected image of the operating arm (31);
generate an animation based on each target projected image; and
play the animation on the display (22) based on a preset frequency.

11. The surgical robot of claim 1, **characterized in that**, when obtaining the virtual camera selected by the input portion, the controller is further configured to:

obtain a motion trace of the virtual camera input by the input portion;
discrete the motion trace to acquire discrete positions of the virtual camera, the discrete positions being target positions of the virtual camera;
determine a target projection point of each of the plurality of feature points in the feature point sequence on a projection plane of the virtual camera at each of the target positions, according to a preset speed of the virtual camera, the kinematic model, and the joint variables;
fit and connect the target projection point of each of the target positions orderly to generate a target projected image of the operating arm (31);
generate an animation based on each of the target projected images; and
play the animation on the display (22) based on a preset frequency.

12. The surgical robot of claim 1, **characterized in that**, the operating arm (31) comprises a camera arm (31a) with an image end instrument (34A), and the controller is configured to:

acquire an operating image of a surgical area captured by the image end instrument (34A);
display the operating image on the display (22);
display the projected image suspended on the operating image;
when displaying the projected image suspended on the operating image, the controller is further configured to:

acquire an overlapping region between the operating image and the projected image, and obtain a first image property of the operating image in the overlapping region;
adjust a second image property of the projected image in the overlapping region based on the first image property.

13. A graphic display method of a surgical robot, wherein the surgical robot comprises:

an input portion;
a display (22);
an operating arm (31) comprising a plurality of joints and a plurality of sensors, the sensor configured to sense joint variables of the plurality of joints, the plurality of joints constituting a locative degree of freedom and/or an orientated degree of freedom, the operating arm (31) further comprising a feature point sequence composed of a plurality of feature points arranged orderly, the plurality of feature points representing the plurality of joints;
wherein the control method comprises:

acquiring the feature point sequence of the operating arm (31) and a kinematic model corresponding to the feature point sequence;

acquiring joint variables sensed by the plurality of sensors, and obtain a virtual camera selected by the input portion, the virtual camera comprises at least one of a virtual focal length and a virtual aperture;

determining a projection point of each of the plurality of feature points in the feature point sequence on a projection plane of the virtual camera according to the kinematic model, the joint variables, and the at least one of the virtual focal length and the virtual aperture;

fitting and connecting the projection point of each of the plurality of feature points orderly to generate a projected image of the operating arm (31);

detecting whether the projected image is distorted;

when the projected image distortion is detected to be distorted, increasing the virtual focal length of the virtual camera, and repeating a process of determining the projection point of each of the plurality of feature points in the feature point sequence on the projection plane of the virtual camera according to the kinematic model, the joint variables, and the at least one of the virtual focal length and the virtual aperture; and

displaying the projected image on the display (22) when the image is detected to be not distorted.

14. A graphical control device of a surgical robot, comprising:

a display (22);
a memory (503) having stored thereon computer programs; and
a processor (501) for loading and executing the computer programs;

wherein the computer programs are configured to be loaded and executed by the processor (501) to perform the graphic display method as claim 13.

**Patentansprüche**

1. Ein chirurgischer Roboter, umfassend:

einen Eingabeteil;
eine Anzeige (22);
einen Operationsarm (31), der eine Vielzahl von Gelenken und eine Vielzahl von Sensoren umfasst, wobei die Vielzahl von Sensoren dazu konfiguriert ist, Gelenkvariablen der Vielzahl von Gelenken zu erfassen, wobei der Operationsarm (31) ferner eine Merkmalspunktsequenz umfasst, die aus einer Vielzahl von Merkmalspunkten zusammengesetzt ist, wobei die Vielzahl von Merkmalspunkten geordnet angeordnet ist und jedes der Vielzahl von Gelenken mit mindestens einem der Vielzahl von Merkmalspunkten assoziiert ist; und

eine Steuerung, die mit jedem von dem Eingabeteil, der Anzeige (22) und der Vielzahl von Sensoren gekoppelt ist, wobei die Steuerung dazu konfiguriert ist:

die Merkmalspunktsequenz des Operationsarms (31) und ein der Merkmalspunktsequenz entsprechendes kinematisches Modell zu erhalten;

die von der Vielzahl von Sensoren erfassten Gelenkvariablen zu erhalten und eine durch den Eingabeteil ausgewählte virtuelle Kamera zu erhalten, wobei die virtuelle Kamera mindestens eines von einer virtuellen Brennweite und einer virtuellen Blende umfasst;

einen Projektionspunkt jedes der Vielzahl von Merkmalspunkten in der Merkmalspunktsequenz auf einer Projektionsebene der virtuellen Kamera gemäß dem kinematischen Modell, den Gelenkvariablen und dem mindestens einen von der virtuellen Brennweite und der virtuellen Blende zu bestimmen;

den Projektionspunkt jedes der Vielzahl von Merkmalspunkten geordnet anzupassen und zu verbinden, um ein projiziertes Bild des Operationsarms (31) zu erzeugen;

zu detektieren, ob das projizierte Bild verzerrt ist;

wenn die Verzerrung des projizierten Bildes als verzerrt detektiert wird, die virtuelle Brennweite der virtuellen Kamera zu erhöhen und einen Prozess des Bestimmens des Projektionspunkts jedes der Vielzahl von Merkmalspunkten in der Merkmalspunktsequenz auf der Projektionsebene der virtuellen Kamera gemäß dem kinematischen Modell, den Gelenkvariablen und dem mindestens einen von der virtuellen Brennweite und der virtuellen Blende zu wiederholen; und

das projizierte Bild auf der Anzeige (22) anzuzeigen, wenn das Bild als nicht verzerrt detektiert wird.

2. Der chirurgischer Roboter nach Anspruch 1, **dadurch gekennzeichnet, dass**,

wenn der Projektionspunkt jedes der Vielzahl von Merkmalspunkten in der Merkmalspunktsequenz auf der Projektionsebene der virtuellen Kamera gemäß dem kinematischen Modell und den Gelenkvariablen bestimmt wird, die Steuerung ferner dazu konfiguriert ist:

eine erste Position jedes der Vielzahl von Merkmalspunkten der Merkmalspunktsequenz in einem Referenzkoordinatensystem auf der Basis des kinematischen Modells und der Gelenkvariablen zu erfassen; die erste Position jedes der Vielzahl von Merkmalspunkten in eine zweite Position in einem Koordinatensystem der virtuellen Kamera umzuwandeln; eine virtuelle Brennweite der virtuellen Kamera zu erfassen und die Projektionsebene der virtuellen Kamera auf der Basis der virtuellen Brennweite zu bestimmen; und einen Projektionspunkt der zweiten Position jedes der Vielzahl von Merkmalspunkten auf der Projektionsebene auf der Basis der virtuellen Brennweite zu erfassen.

3.  Der chirurgischer Roboter nach Anspruch 1, **dadurch gekennzeichnet, dass**,

wenn der Projektionspunkt jedes der Vielzahl von Merkmalspunkten in der Merkmalspunktsequenz auf der Projektionsebene der virtuellen Kamera gemäß dem kinematischen Modell und den Gelenkvariablen bestimmt wird, die Steuerung ferner dazu konfiguriert ist:

eine erste Position jedes der Vielzahl von Merkmalspunkten in einem Referenzkoordinatensystem auf der Basis des kinematischen Modells und der Gelenkvariablen zu erfassen; die erste Position jedes der Vielzahl von Merkmalspunkten in eine zweite Position in einem Koordinatensystem der virtuellen Kamera umzuwandeln; eine Konturinformation jedes der Vielzahl von Gelenken entsprechend den korrespondierenden Merkmalspunkten, die mit dem Gelenk assoziiert sind, zu erfassen; einen Projektionspunkt der zweiten Position jedes der Vielzahl von Merkmalspunkten auf der Projektionsebene gemäß der virtuellen Brennweite und der Konturinformation zu erfassen; und

wenn der Projektionspunkt jedes der Vielzahl von Merkmalspunkten geordnet angepasst und verbunden wird, um das projizierte Bild des Operationsarms (31) zu erzeugen, die Steuerung ferner dazu konfiguriert ist:

den Projektionspunkt jedes der Vielzahl von Merkmalspunkten geordnet anzupassen und zu verbinden, um das projizierte Bild des Operationsarms (31) gemäß der Konturinformation und einer Reihenfolge der Vielzahl von Merkmalspunkten entsprechend den Projektionspunkten der Vielzahl von Merkmalspunkten in der Merkmalspunktsequenz zu erzeugen.

4.  Der chirurgischer Roboter nach Anspruch 1, **dadurch gekennzeichnet, dass**, wenn der Projektionspunkt jedes der Vielzahl von Merkmalspunkten geordnet angepasst und verbunden wird, um das projizierte Bild des Operationsarms (31) zu erzeugen, die Steuerung ferner dazu konfiguriert ist:

einen Typ des Operationsarms (31) zu erfassen und ein Symbol eines Endinstruments (34) des Operationsarms (31) gemäß dem Typ passend zuzuordnen; eine Pose und Position des Endinstruments (34) auf der Projektionsebene der virtuellen Kamera auf der Basis der Gelenkvariablen und des kinematischen Modells zu bestimmen; das Symbol durch Rotieren und/oder Zoomen auf der Basis der Pose und Position des Endinstruments (34) auf der Projektionsebene der virtuellen Kamera zu verarbeiten; und das Symbol mit einem Projektionspunkt, der sich an einem distalen Ende des Operationsarms (31) befindet, zusammenzufügen, um das projizierte Bild zu erzeugen.

5.  Der chirurgischer Roboter nach Anspruch 1, wobei, wenn detektiert wird, ob das projizierte Bild verzerrt ist, die Steuerung dazu konfiguriert ist:

eine Position des Projektionspunkts jedes der Vielzahl von Merkmalspunkten in einem Referenzkoordinatensystem zu erfassen; eine Menge von ersten Projektionspunkten zu erfassen, wobei die ersten Projektionspunkte die Projektionspunkte sind, die in einen Randbereich der Projektionsebene oder einen Randbereich eines Anzeigefensters fallen, wobei das Anzeigefenster dazu konfiguriert ist, das projizierte Bild auf der Anzeige (22) anzuzeigen; und ein Verhältnis der Menge der ersten Projektionspunkte zu einer Gesamtmenge der Projektionspunkte zu berechnen und zu bestimmen, dass das Bild verzerrt ist, wenn das Verhältnis einen Schwellenwert erreicht.

6.  Der chirurgischer Roboter nach Anspruch 1, **dadurch gekennzeichnet, dass**, der Operationsarm (31) einen Kameraarm (31a) mit einem Bild-Endinstrument (34A) umfasst und die Steuerung ferner dazu konfiguriert ist:

einen Kameraparameter des Bild-Endinstruments (34A) des Kameraarms (31a) zu erfassen und einen sichtbaren Bereich des Bild-Endinstruments (34A) gemäß dem Kameraparameter zu berechnen, wobei der Kameraparameter eine Brennweite und eine Blende umfasst;

eine Pose und Position des Bild-Endinstruments (34A) in einem Referenzkoordinatensystem auf der Basis der Gelenkvariablen und des kinematischen Modells des Kameraarms (31a) zu bestimmen;

den sichtbaren Bereich des Bild-Endinstruments (34A) in einen sichtbaren Bereich der virtuellen Kamera auf der Basis einer Umwandlungsbeziehung zwischen der Pose und Position des Bild-Endinstruments (34A) und der Pose und Position der virtuellen Kamera in dem Referenzkoordinatensystem umzuwandeln; und

eine Grenzlinie des sichtbaren Bereichs der virtuellen Kamera auf der Projektionsebene zu berechnen und die Grenzlinie in dem projizierten Bild auf der Anzeige (22) anzuzeigen.

7. Der chirurgischer Roboter nach Anspruch 1, **dadurch gekennzeichnet, dass**,

der Operationsarm (31) einen Kameraarm (31a) mit einem Bild-Endinstrument (34A) und einen chirurgischen Arm (31c) mit einem operativen Endinstrument (34B) umfasst;

wenn der Projektionspunkt jedes der Vielzahl von Merkmalspunkten geordnet angepasst und verbunden wird, um das projizierte Bild des Operationsarms (31) zu erzeugen, die Steuerung ferner dazu konfiguriert ist:

ein Operationsbild eines chirurgischen Bereichs, das von dem Bild-Endinstrument (34A) des Kameraarms (31a) aufgenommen wurde, zu erfassen;

einen Merkmalsteil des chirurgischen Arms (31c) aus dem Operationsbild zu identifizieren; einen assoziierten ersten Merkmalspunkt aus der Merkmalspunktsequenz gemäß dem identifizierten Merkmalsteil passend zuzuordnen; und den Projektionspunkt jedes der Vielzahl von Merkmalspunkten geordnet anzupassen und zu verbinden, einen ersten Projektionspunkt aus den mit dem ersten Merkmalspunkt assoziierten Projektionspunkten zu markieren, und ein mit dem ersten Projektionspunkt verbundenes Liniensegment zu markieren, um das projizierte Bild des Operationsarms (31) zu erzeugen;

wobei die Merkmalspunktsequenz ferner einen nicht zugeordneten zweiten Merkmalspunkt umfasst, wobei nach dem passenden Zuordnen des assoziierten ersten Merkmalspunkts aus den Merkmalspunktsequenzen auf der Basis des identifizierten Merkmalsteils die Steuerung ferner dazu konfiguriert ist:

den nicht zugeordneten zweiten Merkmalspunkt zu erfassen; ein Bildmodell des Merkmalsteils gemäß der Konturinformation, den Gelenkvariablen und dem kinematischen Modell des dem zweiten Merkmalspunkt entsprechenden Merkmalsteils zu erzeugen;

das Bildmodell in ein Ergänzungsbild in einem Koordinatensystem des Bild-Endinstruments (34A) umzuwandeln;

das Ergänzungsbild mit einem Bild des Merkmalsteils, das dem ersten Merkmalspunkt entspricht, auf der Basis der Sequenz des ersten und des zweiten Merkmalspunkts in der Merkmalspunktsequenz zusammenzufügen, um ein vollständiges Teilbild des Operationsarms (31) in dem Operationsbild zu erzeugen; und das Operationsbild mit dem vollständigen Teilbild des Operationsarms (31) auf der Anzeige (22) anzuzeigen.

8. Der chirurgischer Roboter nach Anspruch 1, **dadurch gekennzeichnet, dass** die Steuerung ferner dazu konfiguriert ist:

einen maximalen Bewegungsbereich in einer ersten Ausrichtung des Operationsarms (31) zu erfassen;

einen Bewegungsbetrag in der ersten Ausrichtung des Operationsarms (31) auf der Basis der Gelenkvariablen und des kinematischen Modells zu berechnen;

ein Symbol auf der Basis des maximalen Bewegungsbereichs und des Bewegungsbetrags in der ersten Ausrichtung zu erzeugen; und

das Symbol auf der Anzeige (22) anzuzeigen.

9. Der chirurgischer Roboter nach Anspruch 1, **dadurch gekennzeichnet, dass**,

eine Vielzahl von virtuellen Kameras, die durch den Eingabeteil auswählbar sind, unterschiedliche Posen und Positionen in einem Referenzkoordinatensystem aufweisen; und

die Posen und Positionen der Vielzahl von virtuellen Kameras in dem Referenzkoordinatensystem durch einen erreichbaren Arbeitsraum des Operationsarms (31) in dem Referenzkoordinatensystem bestimmt sind;

die Steuerung ferner dazu konfiguriert ist, das projizierte Bild auf einem ersten Anzeigefenster der Anzeige (22) anzuzeigen und eine Vielzahl von Symbolen der Vielzahl von virtuellen Kameras zu erzeugen.

10. Der chirurgischer Roboter nach Anspruch 1, **dadurch gekennzeichnet, dass**, wenn die durch den Eingabeteil ausgewählte virtuelle Kamera erhalten wird, die Steuerung ferner dazu konfiguriert ist:

die durch den Eingabeteil ausgewählte virtuelle Kamera und mindestens zwei Zielpositionen der virtuellen Kamera, die durch den Eingabeteil eingegeben wurden, zu erhalten; einen Zielprojektionspunkt jedes der Vielzahl von Merkmalspunkten in der Merkmalspunktsequenz auf einer Projektionsebene der virtuellen Kamera an jeder der Zielpositionen gemäß einer voreingestellten Geschwindigkeit der virtuellen Kamera, dem kinematischen Modell und den Gelenkvariablen zu bestimmen; den Zielprojektionspunkt jeder der Zielpositionen geordnet anzupassen und zu verbinden, um ein projiziertes Zielbild des Operationsarms (31) zu erzeugen; eine Animation auf der Basis jedes projizierten Zielbildes zu erzeugen; und die Animation auf der Anzeige (22) auf der Basis einer voreingestellten Frequenz abzuspielen.

11. Der chirurgischer Roboter nach Anspruch 1, **dadurch gekennzeichnet, dass**, wenn die durch den Eingabeteil ausgewählte virtuelle Kamera erhalten wird, die Steuerung ferner dazu konfiguriert ist:

eine Bewegungsspur der virtuellen Kamera, die durch den Eingabeteil eingegeben wurde, zu erhalten; die Bewegungsspur zu diskretisieren, um diskrete Positionen der virtuellen Kamera zu erfassen, wobei die diskreten Positionen Zielpositionen der virtuellen Kamera sind; einen Zielprojektionspunkt jedes der Vielzahl von Merkmalspunkten in der Merkmalspunktsequenz auf einer Projektionsebene der virtuellen Kamera an jeder der Zielpositionen gemäß einer voreingestellten Geschwindigkeit der virtuellen Kamera, dem kinematischen Modell und den Gelenkvariablen zu bestimmen; den Zielprojektionspunkt jeder der Zielpositionen geordnet anzupassen und zu verbinden, um ein projiziertes Zielbild des Operationsarms (31) zu erzeugen; eine Animation auf der Basis jedes der projizierten Zielbilder zu erzeugen; und

die Animation auf der Anzeige (22) auf der Basis einer voreingestellten Frequenz abzuspielen.

12. Der chirurgischer Roboter nach Anspruch 1, **dadurch gekennzeichnet, dass**,

der Operationsarm (31) einen Kameraarm (31a) mit einem Bild-Endinstrument (34A) umfasst und die Steuerung dazu konfiguriert ist:

ein Operationsbild eines chirurgischen Bereichs, das von dem Bild-Endinstrument (34A) aufgenommen wurde, zu erfassen; das Operationsbild auf der Anzeige (22) anzuzeigen; das projizierte Bild schwebend in dem Operationsbild anzuzeigen;

wobei, wenn das projizierte Bild schwebend in dem Operationsbild angezeigt wird, die Steuerung ferner dazu konfiguriert ist: einen Überlappungsbereich zwischen dem Operationsbild und dem projizierten Bild zu erfassen und eine erste Bildeigenschaft des Operationsbildes in dem Überlappungsbereich zu erhalten; eine zweite Bildeigenschaft des projizierten Bildes in dem Überlappungsbereich auf der Basis der ersten Bildeigenschaft anzupassen.

13. Ein grafisches Anzeigeverfahren eines chirurgischen Roboters, wobei der chirurgische Roboter umfasst:

einen Eingabeteil; eine Anzeige (22); einen Operationsarm (31), der eine Vielzahl von Gelenken und eine Vielzahl von Sensoren umfasst, wobei der Sensor dazu konfiguriert ist, Gelenkvariablen der Vielzahl von Gelenken zu erfassen, wobei die Vielzahl von Gelenken einen Ortsfreiheitsgrad und/oder einen Orientierungsfreiheitsgrad bilden, wobei der Operationsarm (31) ferner eine Merkmalspunktsequenz umfasst, die aus einer Vielzahl von geordnet angeordneten Merkmalspunkten zusammengesetzt ist, wobei die Vielzahl von Merkmalspunkten die Vielzahl von Gelenken repräsentiert; wobei das Steuerungsverfahren umfasst:

Erfassen der Merkmalspunktsequenz des Operationsarms (31) und eines kinematischen Modells, das der Merkmalspunktsequenz entspricht; Erfassen von Gelenkvariablen, die von der Vielzahl von Sensoren erfasst werden, und Erhalten einer virtuellen Kamera, die durch den Eingabeteil ausgewählt wird, wobei die virtuelle Kamera mindestens eines von einer virtuellen Brennweite und einer virtuellen Blende umfasst;

Bestimmen eines Projektionspunkts jedes der Vielzahl von Merkmalspunkten in der Merkmalspunktsequenz auf einer Projektionsebene der virtuellen Kamera gemäß dem kinematischen Modell, den Gelenkvariablen und dem mindestens einen von der virtuellen Brennweite und der virtuellen Blende;

Geordnetes Anpassen und Verbinden des Projektionspunkts jedes der Vielzahl von Merkmalspunkten, um ein projiziertes Bild des Operationsarms (31) zu erzeugen;

Detektieren, ob das projizierte Bild verzerrt ist;

wenn die Verzerrung des projizierten Bildes als verzerrt detektiert wird, Erhöhen der virtuellen Brennweite der virtuellen Kamera und Wiederholen eines Prozesses des Bestimmens des Projektionspunkts jedes der Vielzahl von Merkmalspunkten in der Merkmalspunktsequenz auf der Projektionsebene der virtuellen Kamera gemäß dem kinematischen Modell, den Gelenkvariablen und dem mindestens einen von der virtuellen Brennweite und der virtuellen Blende; und

Anzeigen des projizierten Bildes auf der Anzeige (22), wenn das Bild als nicht verzerrt detektiert wird.

14. Eine grafische Steuerungsvorrichtung eines chirurgischen Roboters, umfassend:

eine Anzeige (22);
einen Speicher (503), auf dem Computerprogramme gespeichert sind; und
einen Prozessor (501) zum Laden und Ausführen der Computerprogramme;

wobei die Computerprogramme dazu konfiguriert sind, von dem Prozessor (501) geladen und ausgeführt zu werden, um das grafische Anzeigeverfahren nach Anspruch 13 durchzuführen.

**Revendications**

1. Un robot chirurgical, comprenant:

une partie d'entrée;
un écran (22);
un bras opératoire (31) comprenant une pluralité d'articulations et une pluralité de capteurs, la pluralité de capteurs étant configurée pour détecter des variables d'articulation de la pluralité d'articulations, le bras opératoire (31) comprenant en outre une séquence de points caractéristiques composée d'une pluralité de points caractéristiques, la pluralité de points caractéristiques étant disposée de manière ordonnée, et chacune de la pluralité d'articulations étant associée à au moins l'un de la pluralité de points caractéristiques; et

un contrôleur, couplé à chacun parmi la partie d'entrée, l'écran (22) et la pluralité de capteurs, le contrôleur étant configuré pour:

obtenir la séquence de points caractéristiques du bras opératoire (31) et un modèle cinématique correspondant à la séquence de points caractéristiques;
obtenir les variables d'articulation détectées par la pluralité de capteurs, et obtenir une caméra virtuelle sélectionnée par la partie d'entrée, la caméra virtuelle comprenant au moins l'une parmi une distance focale virtuelle et une ouverture virtuelle;
déterminer un point de projection de chacun de la pluralité de points caractéristiques dans la séquence de points caractéristiques sur un plan de projection de la caméra virtuelle selon le modèle cinématique, les variables d'articulation et ladite au moins une parmi la distance focale virtuelle et l'ouverture virtuelle;
ajuster et relier le point de projection de chacun de la pluralité de points caractéristiques de manière ordonnée pour générer une image projetée du bras opératoire (31);
détecter si l'image projetée est distordue;
lorsque la distorsion de l'image projetée est détectée comme étant distordue, augmenter la distance focale virtuelle de la caméra virtuelle, et répéter un processus de détermination du point de projection de chacun de la pluralité de points caractéristiques dans la séquence de points caractéristiques sur le plan de projection de la caméra virtuelle selon le modèle cinématique, les variables d'articulation et ladite au moins une parmi la distance focale virtuelle et l'ouverture virtuelle; et
afficher l'image projetée sur l'écran (22) lorsque l'image est détectée comme n'étant pas distordue.

2. Le robot chirurgical selon la revendication 1, **caractérisé en ce que**,
lors de la détermination du point de projection de chacun de la pluralité de points caractéristiques dans la séquence de points caractéristiques sur le plan de projection de la caméra virtuelle selon le modèle cinématique et les variables d'articulation, le contrôleur est en outre configuré pour:

acquérir une première position de chacun de la pluralité de points caractéristiques de la séquence de points caractéristiques dans un système de coordonnées de référence sur la base du modèle cinématique et des variables d'articulation;

convertir la première position de chacun de la pluralité de points caractéristiques en une seconde position dans un système de coordonnées de la caméra virtuelle;

acquérir une distance focale virtuelle de la caméra virtuelle et déterminer le plan de projection de la caméra virtuelle sur la base de la distance focale virtuelle; et

acquérir un point de projection de la seconde position de chacun de la pluralité de points caractéristiques sur le plan de projection sur la base de la distance focale virtuelle.

3. Le robot chirurgical selon la revendication 1, **caractérisé en ce que**,

lors de la détermination du point de projection de chacun de la pluralité de points caractéristiques dans la séquence de points caractéristiques sur le plan de projection de la caméra virtuelle selon le modèle cinématique et les variables d'articulation, le contrôleur est en outre configuré pour:

acquérir une première position de chacun de la pluralité de points caractéristiques dans un système de coordonnées de référence sur la base du modèle cinématique et des variables d'articulation;

convertir la première position de chacun de la pluralité de points caractéristiques en une seconde position dans un système de coordonnées de la caméra virtuelle;

acquérir une information de contour de chacune de la pluralité d'articulations correspondant aux points caractéristiques correspondants qui sont associés à l'articulation;

acquérir un point de projection de la seconde position de chacun de la pluralité de points caractéristiques sur le plan de projection selon la distance focale virtuelle et l'information de contour; et

lors de l'ajustement et de la liaison du point de projection de chacun de la pluralité de points caractéristiques de manière ordonnée pour générer l'image projetée du bras opératoire (31), le contrôleur est en outre configuré pour:

ajuster et relier le point de projection de chacun de la pluralité de points caractéristiques de manière ordonnée pour générer l'image projetée du bras opératoire (31) selon l'information de contour et un ordre de la pluralité de points caractéristiques correspondant aux points de projection de la pluralité de points caractéristiques dans la séquence de points caractéristiques.

4. Le robot chirurgical selon la revendication 1, **caractérisé en ce que**,

lors de l'ajustement et de la liaison du point de projection de chacun de la pluralité de points caractéristiques de manière ordonnée pour générer l'image projetée du bras opératoire (31), le contrôleur est en outre configuré pour:

acquérir un type du bras opératoire (31), et faire correspondre une icône d'un instrument d'extrémité (34) du bras opératoire (31) selon le type;

déterminer une pose et une position de l'instrument d'extrémité (34) sur le plan de projection de la caméra virtuelle sur la base des variables d'articulation et du modèle cinématique;

traiter l'icône par rotation et/ou zoom sur la base de la pose et de la position de l'instrument d'extrémité (34) sur le plan de projection de la caméra virtuelle; et

assembler l'icône avec un point de projection situé à une extrémité distale du bras opératoire (31) pour générer l'image projetée.

5. Le robot chirurgical selon la revendication 1, dans lequel lors de la détection de si l'image projetée est distordue, le contrôleur est configuré pour:

acquérir une position du point de projection de chacun de la pluralité de points caractéristiques dans un système de coordonnées de référence;

acquérir une quantité de premiers points de projection, dans lequel les premiers points de projection sont les points de projection qui tombent dans une zone de bord du plan de projection ou une zone de bord d'une fenêtre d'affichage, la fenêtre d'affichage étant configurée pour afficher l'image projetée sur l'écran (22); et calculer un rapport de la quantité des premiers points de projection sur une quantité totale des points de projection, et déterminer que l'image est distordue lorsque le rapport atteint un seuil.

6. Le robot chirurgical selon la revendication 1, **caractérisé en ce que**,

le bras opératoire (31) comprend un bras de caméra (31a) avec un instrument d'extrémité d'image (34A), et le contrôleur est en outre configuré pour:

acquérir un paramètre de caméra de l'instrument d'extrémité d'image (34A) du bras de caméra (31a), et calculer une zone visible de l'instrument d'extrémité d'image (34A) selon le paramètre de caméra, le paramètre de caméra comprenant une distance focale et une ouverture;

déterminer une pose et une position de l'instrument d'extrémité d'image (34A) dans un système de coordonnées de référence sur la base des variables d'articulation et du modèle cinématique du bras de caméra (31a);

convertir la zone visible de l'instrument d'extrémité d'image (34A) en une zone visible de la caméra virtuelle sur la base d'une relation de conversion entre la pose et la position de l'instrument d'extrémité d'image (34A) et la pose et la position de la caméra virtuelle dans le système de coordonnées de référence; et

calculer une ligne de délimitation de la zone visible de la caméra virtuelle sur le plan de projection, et afficher la ligne de délimitation dans l'image projetée sur l'écran (22).

7. Le robot chirurgical selon la revendication 1, **caractérisé en ce que**,

le bras opératoire (31) comprend un bras de caméra (31a) avec un instrument d'extrémité d'image (34A) et un bras chirurgical (31c) avec un instrument d'extrémité opératoire (34B);

lors de l'ajustement et de la liaison du point de projection de chacun de la pluralité de points caractéristiques de manière ordonnée pour générer l'image projetée du bras opératoire (31), le contrôleur est en outre configuré pour:

acquérir une image opératoire d'une zone chirurgicale capturée par l'instrument d'extrémité d'image (34A) du bras de caméra (31a);

identifier une partie caractéristique du bras chirurgical (31c) à partir de l'image opératoire;

faire correspondre un premier point caractéristique associé provenant de la séquence de points caractéristiques selon la partie caractéristique identifiée; et

ajuster et relier le point de projection de chacun de la pluralité de points caractéristiques de manière ordonnée, marquer un premier point de projection parmi les points de projection associés au premier point caractéristique, et marquer un segment de ligne relié au premier point de projection pour générer l'image projetée du bras opératoire (31);

dans lequel la séquence de points caractéristiques comprend en outre un second point caractéristique non correspondant, après avoir fait correspondre le premier point caractéristique associé provenant des séquences de points caractéristiques sur la base de la partie caractéristique identifiée, le contrôleur est en outre configuré pour:

acquérir le second point caractéristique non correspondant;

générer un modèle d'image de la partie caractéristique selon l'information de contour, les variables d'articulation et le modèle cinématique de la partie caractéristique correspondant au second point caractéristique;

convertir le modèle d'image en une image supplémentaire dans un système de coordonnées de l'instrument d'extrémité d'image (34A);

assembler l'image supplémentaire avec une image de la partie caractéristique correspondant au premier point caractéristique sur la base de la séquence des premier et second points caractéristiques dans la séquence de points caractéristiques pour générer une sous-image complète du bras opératoire (31) dans l'image opératoire; et

afficher l'image opératoire avec la sous-image complète du bras opératoire (31) sur l'écran (22).

8. Le robot chirurgical selon la revendication 1, **caractérisé en ce que** le contrôleur est en outre configuré pour:

acquérir une plage de mouvement maximale dans une première orientation du bras opératoire (31);

calculer une quantité de mouvement dans la première orientation du bras opératoire (31) sur la base des variables d'articulation et du modèle cinématique;

générer une icône sur la base de la plage de mouvement maximale et de la quantité de mouvement dans la première orientation; et

afficher l'icône sur l'écran (22).

9. Le robot chirurgical selon la revendication 1, **caractérisé en ce que**,

une pluralité de caméras virtuelles sélectionnables par la partie d'entrée présentent des poses et des positions différentes dans un système de coordonnées de référence; et

les poses et les positions de la pluralité de caméras virtuelles dans le système de coordonnées de référence sont déterminées par un espace de travail atteignable du bras opératoire (31) dans le système de coordonnées de référence;

le contrôleur est en outre configuré pour afficher l'image projetée sur une première fenêtre d'affichage de l'écran (22), et pour générer une pluralité d'icônes de la pluralité de caméras virtuelles.

10. Le robot chirurgical selon la revendication 1, **caractérisé en ce que**,

lors de l'obtention de la caméra virtuelle sélectionnée par la partie d'entrée, le contrôleur est en outre configuré pour:

obtenir la caméra virtuelle sélectionnée par la partie d'entrée et au moins deux positions cibles de la caméra virtuelle entrées par la partie d'entrée;

déterminer un point de projection cible de chacun de la pluralité de points caractéristiques dans la séquence de points caractéristiques sur un plan de projection de la caméra virtuelle à chacune des positions cibles, selon une vitesse prédéfinie de la caméra virtuelle, le modèle cinématique et les variables d'articulation;

ajuster et relier le point de projection cible de chacune des positions cibles de manière ordonnée pour générer une image projetée cible du bras opératoire (31);

générer une animation sur la base de chaque image projetée cible; et

lire l'animation sur l'écran (22) sur la base d'une fréquence prédéfinie.

11. Le robot chirurgical selon la revendication 1, **caractérisé en ce que**,

lors de l'obtention de la caméra virtuelle sélectionnée par la partie d'entrée, le contrôleur est en outre configuré pour:

obtenir une trace de mouvement de la caméra virtuelle entrée par la partie d'entrée;

discrétiser la trace de mouvement pour acquérir des positions discrètes de la caméra virtuelle, les positions discrètes étant des positions cibles de la caméra virtuelle;

déterminer un point de projection cible de chacun de la pluralité de points caractéristiques dans la séquence de points caractéristiques sur un plan de projection de la caméra virtuelle à chacune des positions cibles, selon une vitesse prédéfinie de la caméra virtuelle, le modèle cinématique et les variables d'articulation;

ajuster et relier le point de projection cible de chacune des positions cibles de manière ordonnée pour générer une image projetée cible du bras opératoire (31);

générer une animation sur la base de chacune des images projetées cibles; et

lire l'animation sur l'écran (22) sur la base d'une fréquence prédéfinie.

12. Le robot chirurgical selon la revendication 1, **caractérisé en ce que**,

le bras opératoire (31) comprend un bras de caméra (31a) avec un instrument d'extrémité d'image (34A), et le contrôleur est configuré pour:

acquérir une image opératoire d'une zone chirurgicale capturée par l'instrument d'extrémité d'image (34A);

afficher l'image opératoire sur l'écran (22);

afficher l'image projetée en suspension sur l'image opératoire;

lors de l'affichage de l'image projetée en suspension sur l'image opératoire, le contrôleur est en outre configuré pour:

acquérir une région de chevauchement entre l'image opératoire et l'image projetée, et obtenir une première propriété d'image de l'image opératoire dans la région de chevauchement;

ajuster une seconde propriété d'image de l'image projetée dans la région de chevauchement sur la base de la première propriété d'image.

13. Un procédé d'affichage graphique d'un robot chirurgical, dans lequel le robot chirurgical comprend:

une partie d'entrée;

un écran (22);

un bras opératoire (31) comprenant une pluralité d'articulations et une pluralité de capteurs, le capteur étant configuré pour détecter des variables d'articulation de la pluralité d'articulations, la pluralité d'articulations constituant un degré de liberté de localisation et/ou un degré de liberté d'orientation, le bras opératoire (31) comprenant en outre une séquence de points caractéristiques composée d'une pluralité de points caractéristiques disposés de manière ordonnée, la pluralité de points caractéristiques représentant la pluralité d'articulations;

dans lequel le procédé de commande comprend:

l'acquisition de la séquence de points caractéristiques du bras opératoire (31) et d'un modèle cinématique correspondant à la séquence de points caractéristiques;

l'acquisition de variables d'articulation détectées par la pluralité de capteurs, et l'obtention d'une caméra virtuelle sélectionnée par la partie d'entrée, la caméra virtuelle comprenant au moins l'une parmi une distance focale virtuelle et une ouverture virtuelle;

la détermination d'un point de projection de chacun de la pluralité de points caractéristiques dans la séquence de points caractéristiques sur un plan de projection de la caméra virtuelle selon le modèle cinématique, les variables d'articulation et ladite au moins une parmi la distance focale virtuelle et l'ouverture virtuelle;

l'ajustement et la liaison du point de projection de chacun de la pluralité de points caractéristiques de manière ordonnée pour générer une image projetée du bras opératoire (31);

la détection de si l'image projetée est distordue;

lorsque la distorsion de l'image projetée est détectée comme étant distordue, l'augmentation de la distance focale virtuelle de la caméra virtuelle, et la répétition d'un processus de détermination du point de projection de chacun de la pluralité de points caractéristiques dans la séquence de points caractéristiques sur le plan de projection de la caméra virtuelle selon le modèle cinématique, les variables d'articulation et ladite au moins une parmi la distance focale virtuelle et l'ouverture virtuelle; et

l'affichage de l'image projetée sur l'écran (22) lorsque l'image est détectée comme n'étant pas distordue.

14. Un dispositif de commande graphique d'un robot chirurgical, comprenant:

un écran (22);
une mémoire (503) sur laquelle sont stockés des programmes informatiques; et
un processeur (501) pour charger et exécuter les programmes informatiques;

dans lequel les programmes informatiques sont configurés pour être chargés et exécutés par le processeur (501) pour mettre en œuvre le procédé d'affichage graphique selon la revendication 13.

FIG. 1

FIG. 2

FIG. 3

| acquiring the feature point sequence of the operating arm and the kinematic model corresponding to the operating arm | S11 |

| acquiring joint variables sensed by the sensors of each joint of the operating arm | S12 |

| acquiring the virtual camera selected by the input portion | S13 |

| determining a projection point of each of the plurality of feature points in the feature point sequence on a projected image of the virtual camera according to the kinematic model and the joint variables | S14 |

| fitting and connecting the projection point of each of the plurality of feature points orderly to generate a projected image of the operating arm | S15 |

| displaying the projected image on the display | S16 |

FIG.4

FIG.5

FIG. 6

Please select a
virtual camera

Please set a virtual
focal length

f=17mm

f=35mm

f=50mm

setting a
vitrual aperture

F2.8

F4

F5.6

F8

FIG. 7

projection
plane

q1

q2

q3

q4

virtual
camera

Q1

Q2

Q3

Q4

FIG. 8

FIG. 9

FIG. 10

```
┌─────────────────────────────────────────────────────────────────────┐
│        acquiring the icon of the end instrument of the operating arm  │──  S151
└─────────────────────────────────────────────────────────────────────┘
                                    ↓
┌─────────────────────────────────────────────────────────────────────┐
│  determining the pose and position of the end instrument at the       │
│  coordinate system of the                                             │──  S152
│  virtual camera according to the joint variables and the kinematics   │
│  model                                                                │
└─────────────────────────────────────────────────────────────────────┘
                                    ↓
┌─────────────────────────────────────────────────────────────────────┐
│  processing the icon by rotating and/or zooming according to the pose │
│  and position of the                                                  │──  S153
│  end instrument at the coordinate system of the virtual camera        │
└─────────────────────────────────────────────────────────────────────┘
                                    ↓
┌─────────────────────────────────────────────────────────────────────┐
│  splicing the icon with one projection point located at a distal end  │
│  of the operating arm to                                              │──  S154
│  generate a projected image                                           │
└─────────────────────────────────────────────────────────────────────┘
```

FIG. 11

FIG. 12

FIG. 13

| detecting whether there is a camera arm in the operating arm | S21 |

↓

| acquiring the camera parameters of the image end instrument of the camera arm, and calculating the visible area of the image end instrument according to the camera parameters | S22 |

↓

| determining the pose and position of the image end instrument at the reference coordinate system according to the joint variables and the kinematics model of the camera arm | S23 |

↓

| converting the visible area of the image end instrument to be the visible area of the virtual camera based on the conversion relationship between the pose of the image end instrument and the pose of the virtual camera at the reference coordinate system | S24 |

↓

| calculating the boundary line of the visible area on the projection plane of the virtual camera, and display the boundary line in the projected image displayed on the display | S25 |

FIG. 14

FIG. 15

| | |
|---|---|
| acquiring the operation image of the operation area captured by the image end instrument of the camera arm | S151' |
| identifying the feature portion of the operating arm from the operating image | S152' |
| matching out the associated first feature point from the feature point sequence according to the identified feature portion | S153' |
| fitting and connecting the projection point of each of the plurality of feature points orderly, marking the first projection point of the projection points associated with the first feature point, and marking a line segment connected to the first projection point to generate the projected image of the operating arm | S154' |

FIG. 16

acquiring the unmatched second feature point | S155'

generating an image model of the corresponding feature portion according to the contour information, the joint variables, and the kinematic model of the feature portion corresponding to the second feature point | S156'

converting the image model to a supplementary image at the coordinate system of the image end instrument | S157'

splicing the supplementary image with an image of the feature portion corresponding to the first feature point based on the sequence of the first and second feature points in the feature point sequence to generate a complete sub-image of the operating arm in the operating image | S158'

displaying the operating image with the complete sub-image of the operating arm on the display | S159'

FIG.17

FIG. 18

obtaining the maximum range of motion in the first orientation of the operating arm — S31

calculating the amount of the motion in the first orientation of the operating arm according to the joint variables and the kinematics model — S32

generating an icon according to the maximum motion range of motion and the amount of the motion in the first orientation — S33

displaying the icon on the display — S34

FIG. 19

FIG. 20

FIG. 21

obtaining the virtual camera selected by the input unit and at least two target positions of the virtual camera input by the input unit — S131

determining the target projection point of each of the plurality of feature points in the feature point sequence on the projection plane of the virtual camera at each of the target position, according to a preset motion speed of the virtual camera , the kinematic model and the joint variables — S132

fitting and connecting the target projection point of each of the target positions orderly to generate a target projected image of the operating arm — S133

generating an animation according to each target projected image — S134

playing the animation on the display according to the preset frequency — S135

FIG. 22

acquiring the motion trace of the virtual camera input by the input portion — S1311'

discretizing motion trace to obtain discrete positions of the virtual camera, the discrete positions being target positions of the virtual camera — S1312'

determining the target projection point of each of the plurality of feature points in the feature point sequence on the projection plane of the virtual camera at each of the target positions, according to the preset motion speed of the virtual camera, the kinematic model and the joint variables — S132

fitting and connecting the target projection point of each of the target positions orderly to generate a target projected image of the operating arm — S133

generating an animation according to each of the target projected images — S134

playing the animation on the display according to the preset frequency — S135

FIG. 23

Please set a trace of the
virtual camera

FIG. 24

acquiring the operating image of the surgical area collected by the image end instrument — S41

displaying the operating image on the display — S42

displaying the projected image suspended in the operating image — S43

FIG. 25

acquiring the overlapping region between the operating image and the projected image, and acquiring the first image property of the operating image in the overlapping region — S431

adjusting the second image property of the projected image in the overlapping region according to the first image property — S432

FIG. 26

determining the projection point of each of the plurality of feature points in the feature point sequence on the projection plane of the virtual camera according to the virtual focal length and/or virtual aperture, the kinematic model and the joint variables — S14'

fitting and connecting the projection points of each of the plurality of feature points orderly to generate a projected image of the operating arm — S15

S161

whether the projected image is distorted

yes

increasing the virtual focal length of the virtual camera — S162

S16

displaying the projected image on the display

FIG. 27

FIG. 28

FIG. 29

FIG. 30

FIG. 31

FIG. 32

FIG. 33

FIG. 34

constructing respective geometric models of the corresponding first and the second operating arms according to the respective kinematic models and structural features of the first and the second operating arm — S511

discretizing the respective geometric models of the first operating arm and the second operating arm to obtain the respective external information point sets of the first operating arm and the second operating arm at the reference coordinate system — S512

determining the minimum distance between the first operating arm and the second operating arm according to the respective external information point sets of the first operating arm and the second operating arm — S513

FIG. 35

determining the minimum distance point on the projected image of the first operating arm and the second operating arm corresponding to the minimum distance between the first operating arm and the second operating arm — S531

performing a first identification on the minimum distance point on the projected images of the first operating arm and the second operating arm — S532

FIG. 36

determining the collision orientation according to the position of the minimum distance point on the projected images of the first operating arm and the second operating arm at the reference coordinate system — S533

marking the collision orientation between the first operating arm and the second operating arm in the projected image — S534

FIG. 37

determining the collision orientation according to the position of the minimum distance point on the projected images of the first operating arm and the second operating arm at the reference coordinate system — S533

generating a resistance that prevents the mechanical handle from moving in the associated orientation according to the collision orientation — S535

FIG. 38

EP 4 218 652 B1

FIG. 39

FIG. 40

46

## REFERENCES CITED IN THE DESCRIPTION

**Patent documents cited in the description**

- CN 202011068091
- EP 3115159 A1
- WO 2010151438 A1